(19) Europäisches Patentamt
European Patent Office
Office européen des brevets

(11) **EP 2 994 756 B1**

(12) **EUROPEAN PATENT SPECIFICATION**

(45) Date of publication and mention
of the grant of the patent:
**20.06.2018  Bulletin 2018/25**

(51) Int Cl.:
***G01N 33/569*** (2006.01)

(21) Application number: **13834382.7**

(22) Date of filing: **09.05.2013**

(86) International application number:
**PCT/CN2013/075406**

(87) International publication number:
**WO 2014/179965 (13.11.2014 Gazette 2014/46)**

(54) **BIOMARKER IDENTIFYING METHOD AND SYSTEM**

VERFAHREN UND SYSTEM ZUR BIOMARKERIDENTIFIZIERUNG

PROCÉDÉ ET SYSTÈME D'IDENTIFICATION D'UN MARQUEUR BIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.03.2016  Bulletin 2016/11**

(73) Proprietor: **The Procter & Gamble Company Cincinnati, OH 45202 (US)**

(72) Inventors:
• **LI, Rui
  Beijing 101312 (CN)**
• **HUANG, Shi
  Qingdao
  Shandong 266101 (CN)**
• **HE, Tao
  Cincinnati, Ohio 45202 (US)**
• **LIU, Jiquan
  Singapore 138648 (SG)**
• **XU, Jian
  Qingdao
  Shandong 266101 (CN)**

(74) Representative: **Joos, Uta Susanne et al
Procter & Gamble Service GmbH
IP Department
Sulzbacher Strasse 40 - 50
65824 Schwalbach am Taunus (DE)**

(56) References cited:
**WO-A1-2011/011094    WO-A1-2014/019267**

CN-A- 102 517 392

• ANA PAULA V. COLOMBO ET AL: "Impact of Periodontal Therapy on the Subgingival Microbiota of Severe Periodontitis: Comparison Between Good Responders and Individuals With Refractory Periodontitis Using the Human Oral Microbe Identification Microarray", JOURNAL OF PERIODONTOLOGY, vol. 83, no. 10, 1 October 2012 (2012-10-01), pages 1279-1287, XP055205034, ISSN: 0022-3492, DOI: 10.1902/jop.2012.110566
• THERESIA LAKSMANA ET AL: "Metagenomic Analysis of Subgingival Microbiota Following Non-Surgical Periodontal Therapy: a Pilot Study", THE OPEN DENTISTRY JOURNAL, vol. 6, no. 1, 28 December 2012 (2012-12-28), pages 255-261, XP55205077, ISSN: 1874-2106, DOI: 10.2174/1874210601206010255
• XIAO ZHU ET AL: "Possible variation of the human oral bacterial community after wearing removable partial dentures by DGGE", WORLD JOURNAL OF MICROBIOLOGY AND BIOTECHNOLOGY, KLUWER ACADEMIC PUBLISHERS, DO, vol. 28, no. 5, 6 March 2012 (2012-03-06), pages 2229-2236, XP035045401, ISSN: 1573-0972, DOI: 10.1007/S11274-012-1030-5
• JUTTA ZWIELEHNER ET AL: "Changes in Human Fecal Microbiota Due to Chemotherapy Analyzed by TaqMan-PCR, 454 Sequencing and PCR-DGGE Fingerprinting", PLOS ONE, vol. 6, no. 12, 14 December 2011 (2011-12-14), page e28654, XP055110348, ISSN: 1932-6203, DOI: 10.1371/journal.pone.0028654

**Description**

FIELD OF THE INVENTION

The present invention relates to a method of identifying a biomarker indicative of a subject mammal's condition. The present invention also relates to a computer-aided system of identifying a biomarker indicative of a subject mammal's condition.

BACKGROUND OF THE INVENTION

Health condition of a subject is customarily evaluated on the basis of a variety of symptoms. However, many of the symptoms used today, because of their subjective description and uncertain relationship to the disease state, are misleading.

A term "biomarker (biological marker)" was introduced in 1989 as a Medical Subject Heading (MeSH) term and defined as a characteristic that is objectively measured and evaluated as an indicator of normal biological processes, pathogenic processes, or pharmacologic responses to a therapeutic intervention. Biomarker discovery has grown dramatically during the past decades. Biomarkers play major roles in medicinal biology. Biomarkers help in early diagnosis, disease prevention, drug target identification, drug response etc. Several biomarkers have been identified for many diseases such as serum LDL for cholesterol, blood pressure, P53 gene (Loukopoulos P, Thornton JR, Robinson WF (May 2003). "Clinical and pathologic relevance of p53 index in canine osseous tumors". Vet. Pathol. 40 (3): 237-48. doi:10.1354/vp.40-3-237) and MMPs (Loukopoulos P, Mungall BA, Straw RC, Thornton JR, Robinson WF (July 2003). "Matrix metalloproteinase-2 and -9 involvement in canine tumors". Vet. Pathol. 40 (4): 382-94. doi:10.1354/vp.40-4-382) for cancer etc. Introduction of DNA microarrays in the mid-1990s enabled a revolution in transcriptomics and triggered a major paradigm shift in the way life scientists approached research. Subsequently, metabolomics and metabonomics, applied mainly to safety-related biomarkers originally, began to turn to disease-related biomarkers.

Scientists continue their effort in finding new biomarkers that are more closely linked to the underlying causes of health or disease. Their discoveries are set to transform the practice of medicine by giving doctors a more objective and quantifiable basis for clinical decision-making. The microbial community found in oral cavity, the structure or function of which varies with disease progression, offers one of the most promising leads.

The oral cavity is a major site for microbial colonization. Oral microbial community varies among different individuals, different locations within the same oral cavity, or same location at different points in time. The differences in microbial community determine the balance of oral microbial ecosystem, which is directly associated with oral health status and even overall systemic health status. The discovery of biomarkers, however, which must be selected from tens of thousands of microbial types in the microbial community, presents a challenge.

Gingivitis, which involves inflammation of the soft tissues surrounding the teeth, is one of the most prevalent infections and the most common oral disease in humans. As a worldwide health concern, it affects most children and adolescents. The disease is believed to be a result from build-up of plaque and ensuing interactions between the plaque microbiota and host tissues. Although no apical migration of the junctional epithelium occurs, these tissues become erythematous and bleed upon probing. Moreover, chronic gingivitis can progress to periodontitis, which is an irreversible periodontal infection characterized by alveolar bone loss, attachment loss, formation of periodontal pockets, and eventually tooth loss. Therefore, preventive measures against gingivitis, and improved tools for prognosis and early diagnosis thereof, are of particular clinical significance.

Several factors have hindered investigation of the etiology of gingivitis, which remains poorly understood. In natural human populations, gingivitis symptoms can be reversible and volatile, as numerous internal or external factors, including oral hygiene practices (personal or professional), impairment of immune system, injury, diet and oral state, may all potentially affect disease development, thereby confounding disease monitoring. Moreover, clinical diagnosis of gingivitis is based on individual observations and judgment by human examiners. Consequently, the results can be difficult to compare between different patients and different examiners. Furthermore, despite the complexity of oral microbial communities and the suspected polymicrobial nature of chronic oral infections, population-wide surveys of gingivitis-associated microbiota have usually been limited to only a few culturable bacteria (e.g. the "red complex" including *Porphyromonas gingivalis, Tannerella forsythia,* and *Treponema denticola*), which provide insufficient data points for a thorough analysis of various microbes that may potentially cause gingivitis. Ana Paula V. Colombo et al. "Impact of Periodontal Therapy on the Subgingival Microbiota of Severe Periodontitis: Comparison Between Good Responders and Individuals With Refractory Periodontitis Using the Human Oral Microbe Identification Microarrray"; Journal of Periodontology, vol. 83, no. 10, (2012-10-01), pages 1279-1287 discloses a method to investigate bacterial species associated with

periodontitis. Patients with periodontitis were sampled, then treated with surgery and antibiotics. Patients that responded well were resampled when they were cured and patients that did not respond well were sample earlier.

Samples were analyzed and bacterial species associated with disease persistence were found. CN 102 517 392 A and WO 2011/011094 A1 disclose apparatus for analyzing microbial communities including sequencing an analyzing the DNA of the microbioal samples.

Accordingly, there continues to be a need for improved diagnostic methods for assessing the health condition of a subject. There continues to be a need for investigating the etiology of a disease. There continues to be a need for identifying biomarkers which can serve as more sensitive, reliable and objective measures of a disease. There continues to be a need for accurate determination of a subject's susceptibility to a disease so as to prevent and control undesirable conditions and diseases.

SUMMARY OF THE INVENTION

To address these challenges and/or needs, a retrogression-progression model (RPM) has been designed, in combination with analysis of oral microbial community, to investigate the etiology of a disease.

In one aspect, the present invention relates to a method of identifying a biomarker indicative of a subject mammal's condition, comprising the steps:

a) selecting a first set of test mammals having a first disease;

b) obtaining a first oral sample containing a first microbial community from each of the first set of test mammals having the first disease, wherein the first microbial community comprises one or more microbial types;

c) treating each of the first set of test mammals having the first disease, who have been first oral sampled, so as to eliminate or reduce the first disease;

d) obtaining a second oral sample containing a second microbial community from each of the first set of test mammals who have been treated, wherein the second microbial community comprises one or more microbial types;

e) making the first disease reoccur in each of the first set of test mammals who have been second oral sampled;

f) obtaining a third oral sample containing a third microbial community from each of the first set of test mammals in whom the first disease has reoccurred, wherein the third microbial community comprises one or more microbial types;

g) measuring the first, second and third oral samples to obtain abundances of the one or more microbial types in the first, second and third microbial communities, respectively;

h) statistically analyzing the obtained abundances of the one or more microbial types in the first, second and third microbial communities across the first set of test mammals to identify those microbial types whose abundances correlate with a statistical significance to a condition of the first set of test mammals as a first group of microbial types, wherein the condition is selected from the group consisting of: presence of the first disease, severity of the first disease, sensitivity to the first disease, and combinations thereof;

i) selecting one or more microbial types from the first group of microbial types as the biomarker indicative of said subject mammal's condition.

In another aspect, the present invention relates to a computer-aided system of identifying a biomarker indicative of a subject mammal's condition, comprising:

a) a sampling section for sampling:

1) a first oral sample containing a first microbial community from each of a set of test mammals having a disease, wherein the first microbial community comprises one or more microbial types,

2) a second oral sample containing a second microbial community from each of the set of test mammals who have been treated to eliminate or reduce the disease, wherein the second microbial community comprises one or more microbial types, and

3) a third oral sample containing a third microbial community from each of the set of test mammals in whom the disease has reoccurred, wherein the third microbial community comprises one or more microbial types;

b) a measuring section in communication with the sampling section, wherein the measuring section is configured for measuring the first, second and third oral samples to obtain abundances of the one or more microbial types in the first, second and third microbial communities, respectively; and

c) a computing section in communication with the measuring section, wherein the computing section is configured for receiving and statistically analyzing the obtained abundances of the one or more microbial types in the first, second and third microbial communities across the set of test mammals to identify those microbial types whose abundances correlate with a statistical significance to a condition of the set of test mammals as the biomarker indicative of said subject mammal's condition,

wherein the condition is selected from the group consisting of: presence of the disease, severity of the disease, sensitivity to the disease, and combinations thereof.

The present method and system are based on the concept that a balanced oral environment is an indicator of ideal oral health and hygiene status, specifically in terms of a balance in the microbial community. The present invention is achieved by the combination of RPM and oral microbial community analysis. RPM is a retrogression-progression model including two segments, namely, a first segment from a diseased status to a healthy status and a second segment from a healthy status to a reoccurring diseased status. Microbial community analysis has in the past been limited in only one stage, for example from diseased status to healthy status or from healthy status to diseased status. By combining RPM and oral microbial community analysis, the present invention provides an effective way to identify a biomarker which can serve as an objective, reproducible and sensitive measure of health condition.

These and other features, aspects, and advantages of the present invention will become evident to those skilled in the art from the detailed description which follows.

BRIEF DESCRIPTION OF THE DRAWINGS

While the specification concludes with claims particularly defining and distinctly claiming the invention, it is believed that the invention will be better understood from the following description of the accompanying figures. In the accompanying figures,

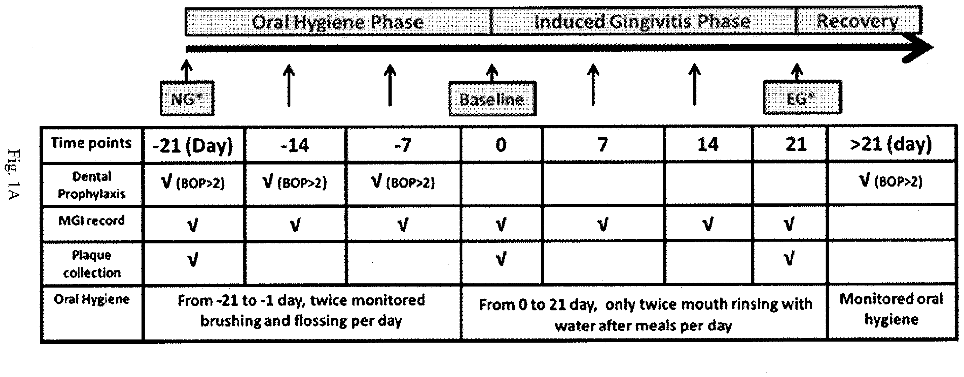

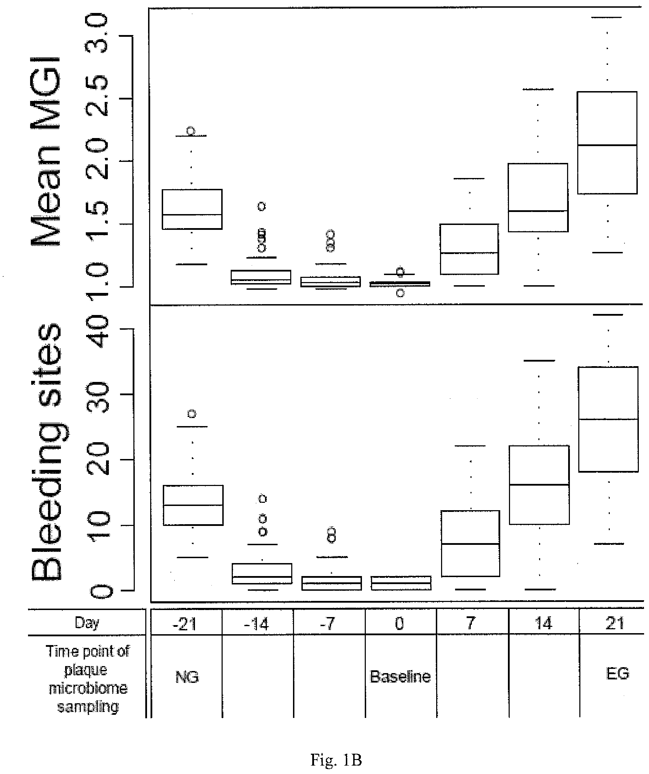

Fig. 1A illustrates a design of longitudinal study simulating gingivitis development in human population according to a specific embodiment of the present invention. Fig. 1B shows values of certain clinical parameters for 50 subjects throughout the study at different time points.

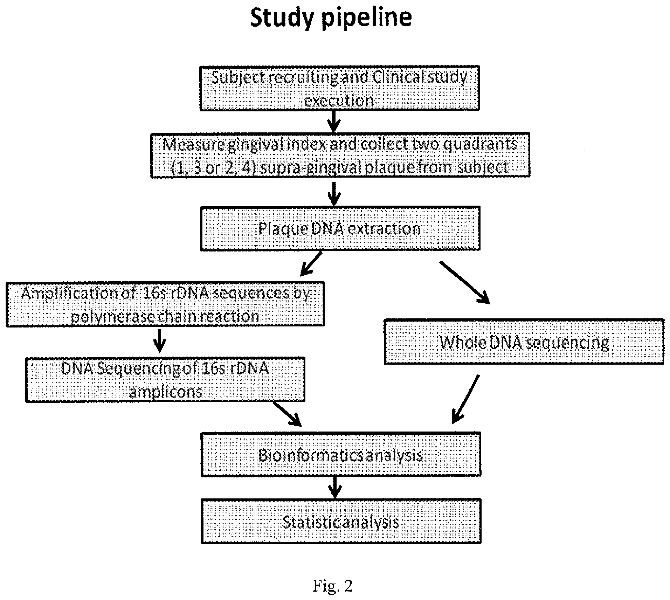

Fig. 2 shows a study pipeline of the method according to a specific embodiment of the present invention.

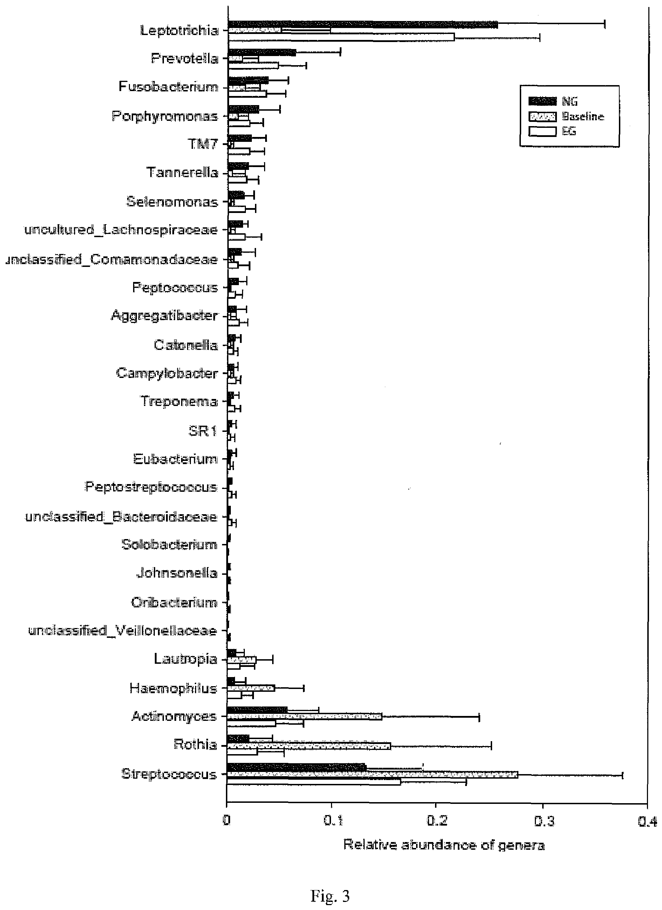

Fig. 3 shows the abundances of 27 genus-level bacterial biomarkers that distinguish between a healthy state and gingivital state(s) (including both naturally occurring gingivitis state and experimentally induced gingivitis state) in 50 subjects, according to a specific embodiment of the present invention.

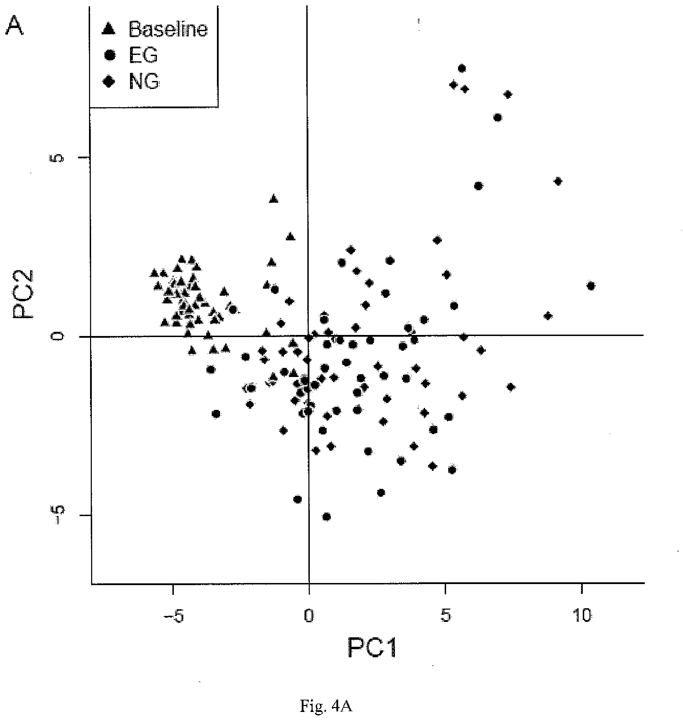

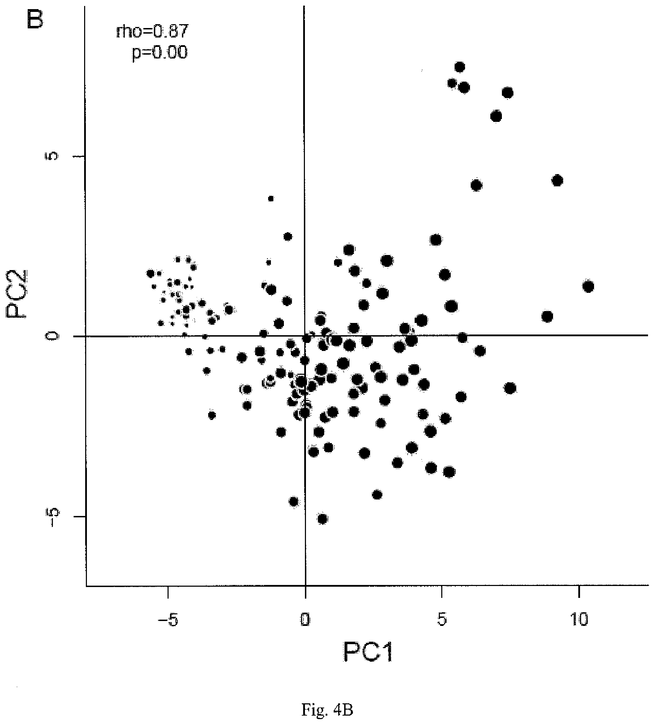

Figs. 4A and 4B show plots of principal components 1 and 2 (PC1 and PC2) from a principal component analysis (PCA) of genus-level bacteria data measured for 150 oral cavity samples collected from 50 subjects at three different stages, i.e., a naturally occurring gingivital stage ("NG"), a baseline stage ("Baseline"), and an experimentally induced gingivital stage ("EG"), according to a specific embodiment of the present invention.

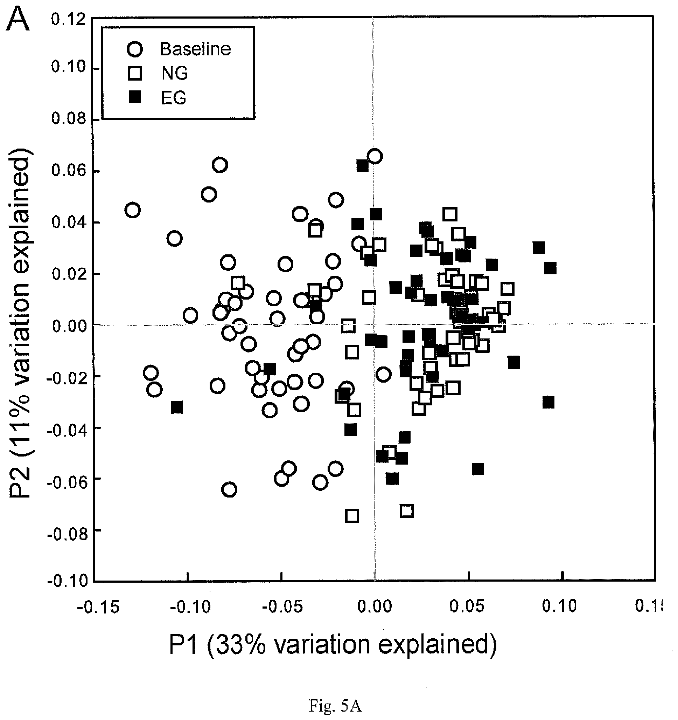

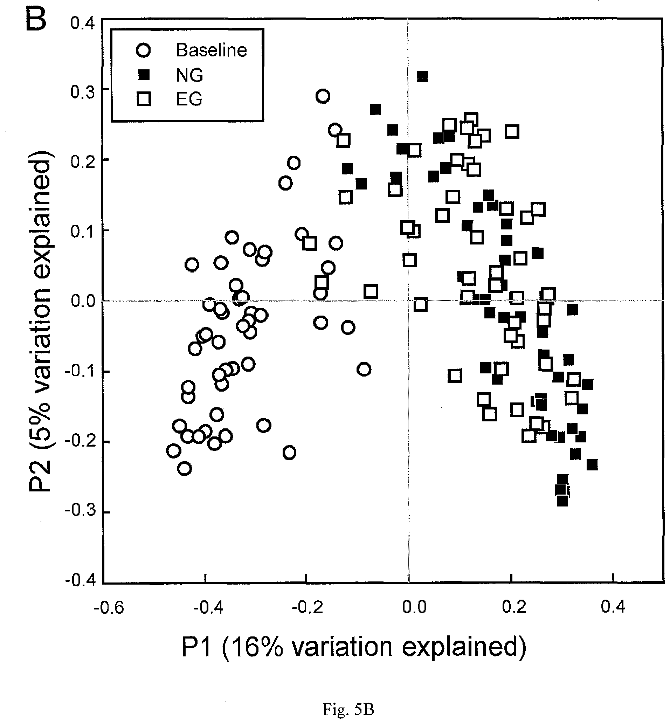

Figs. 5A and 5B show principal coordinate analysis (PCoA) of organismal structures of plaque microbiota according to a specific embodiment of the present invention. Each point corresponds to a microbial community. Fig. 5A is based on the UniFrac distance; and Fig. 5B is based on the thetaYC distance.

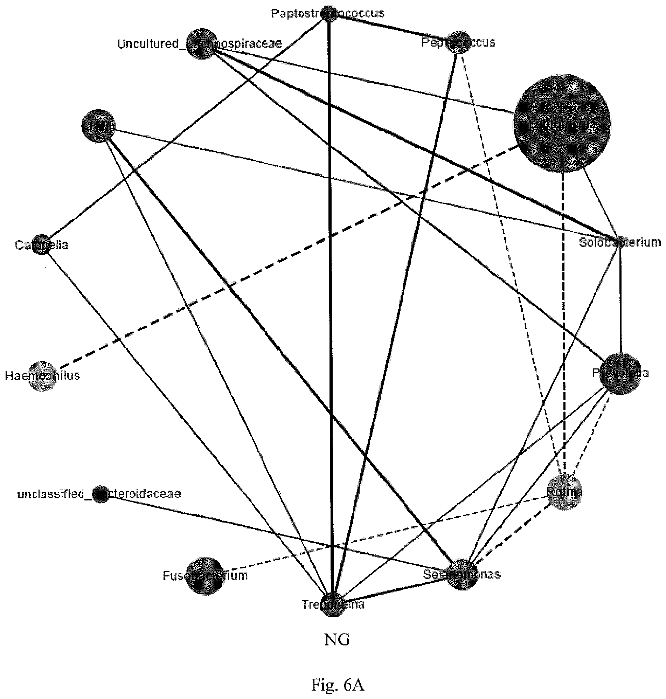

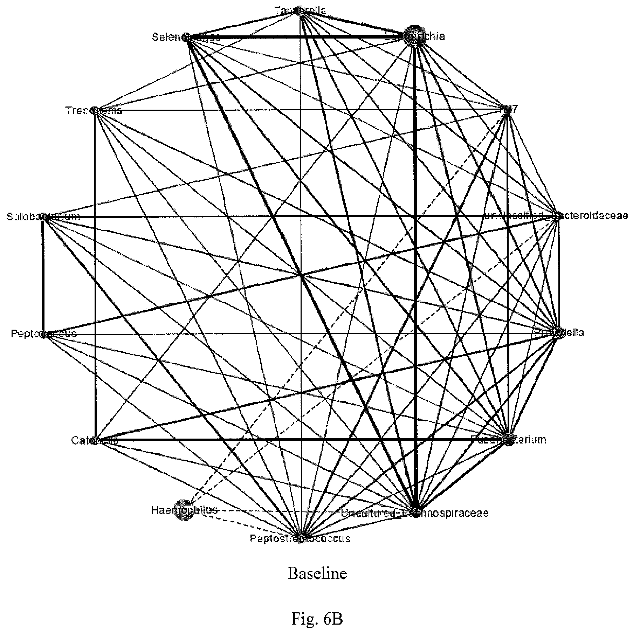

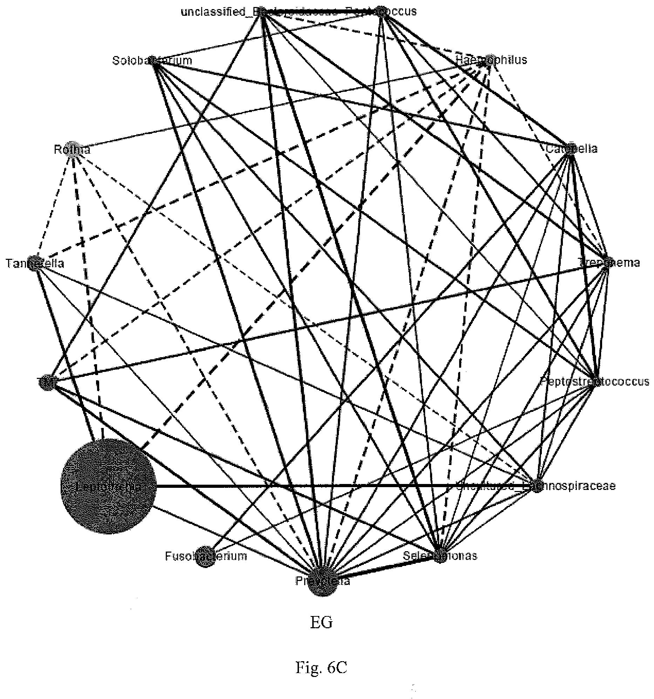

Fig. 6A, 6B and 6C are correlation networks showing interactions among 15 driver genera of gingivitis identified through PCA analysis according to a specific embodiment of the present invention.

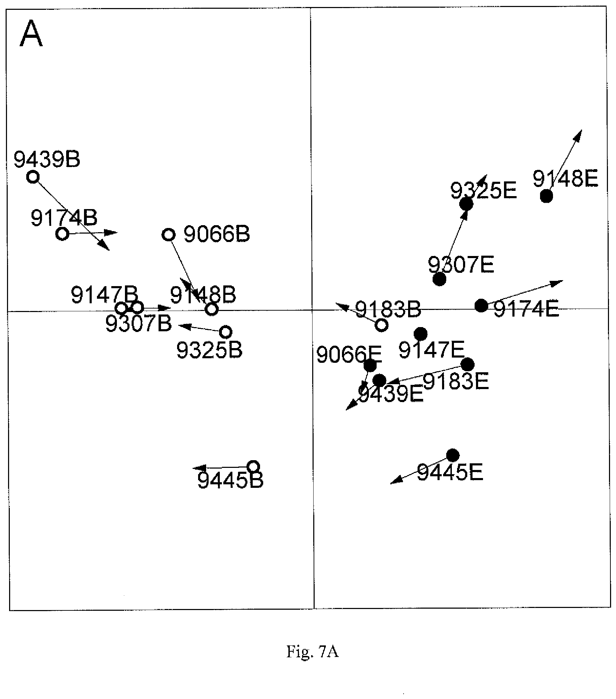

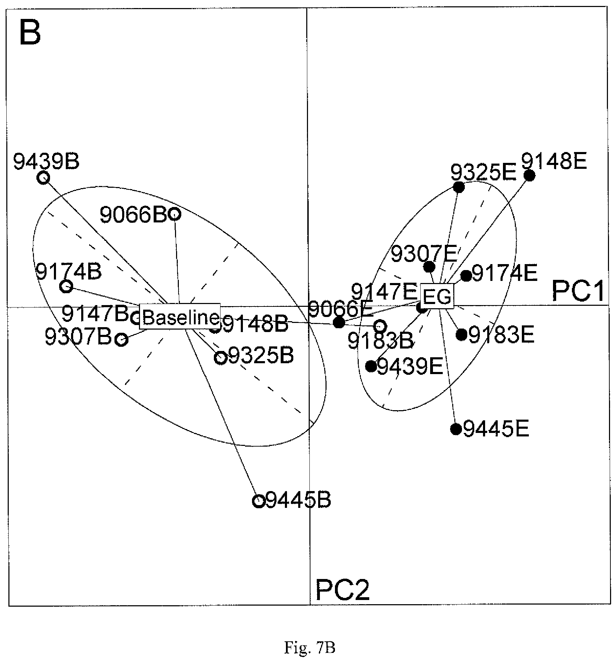

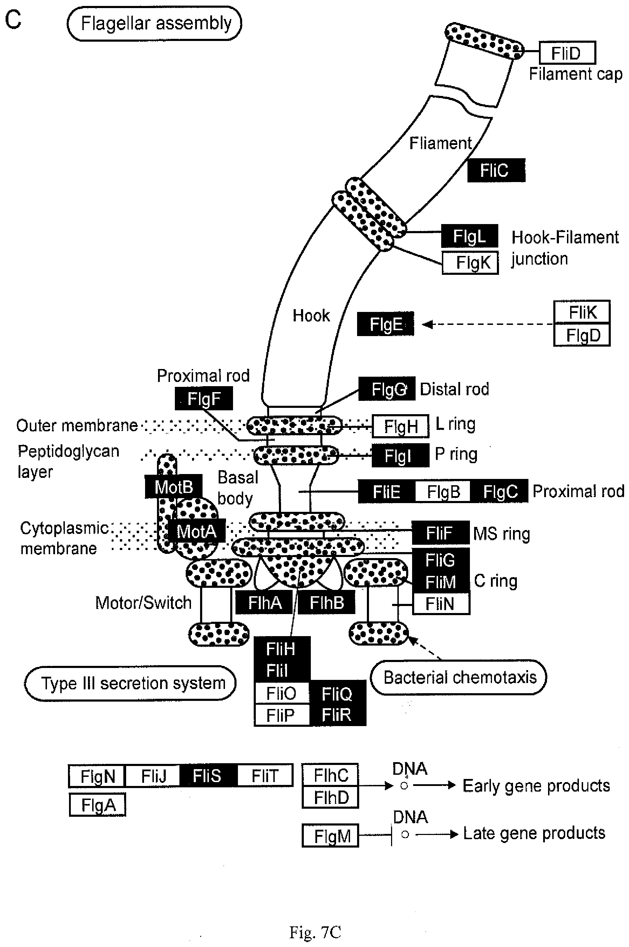

Fig. 7A shows functional distinctions between healthy and Gingivitis microbiota; Fig. 7B shows procrustes analysis of 16S rRNA gene sequences against Clusters of Orthologous Groups (COG); and Fig. 7C shows the 33 gingivitis-enriched orthologous groups (OG) that encode components of the flagellar biosynthesis pathway.

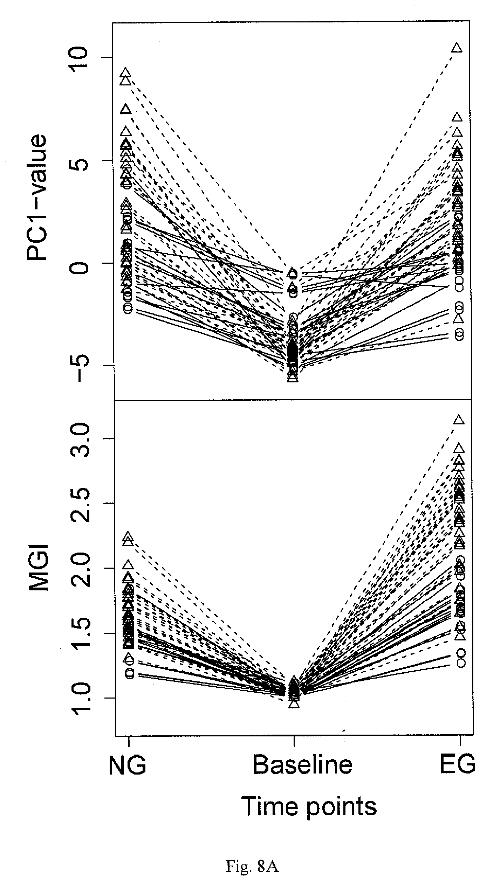

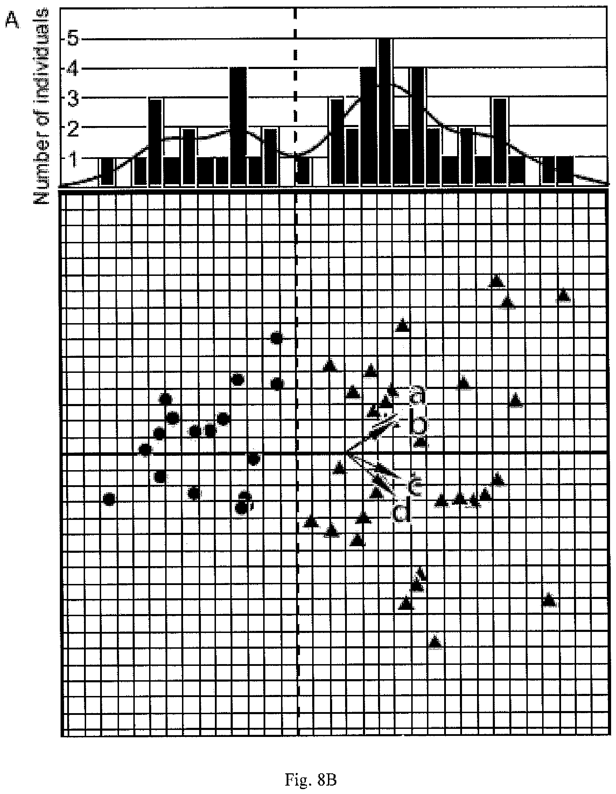

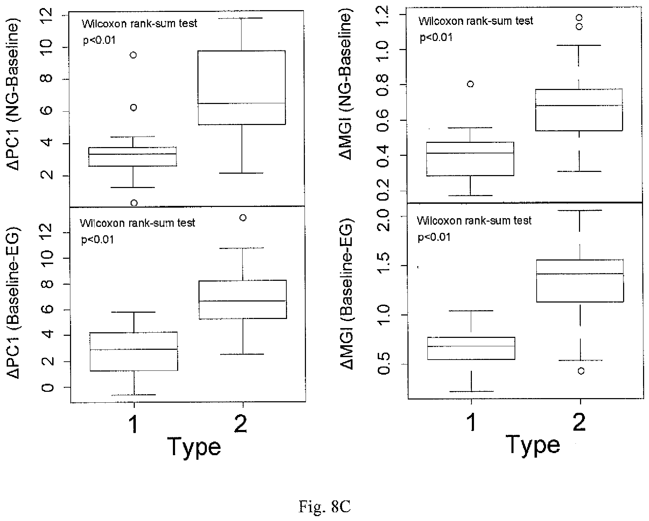

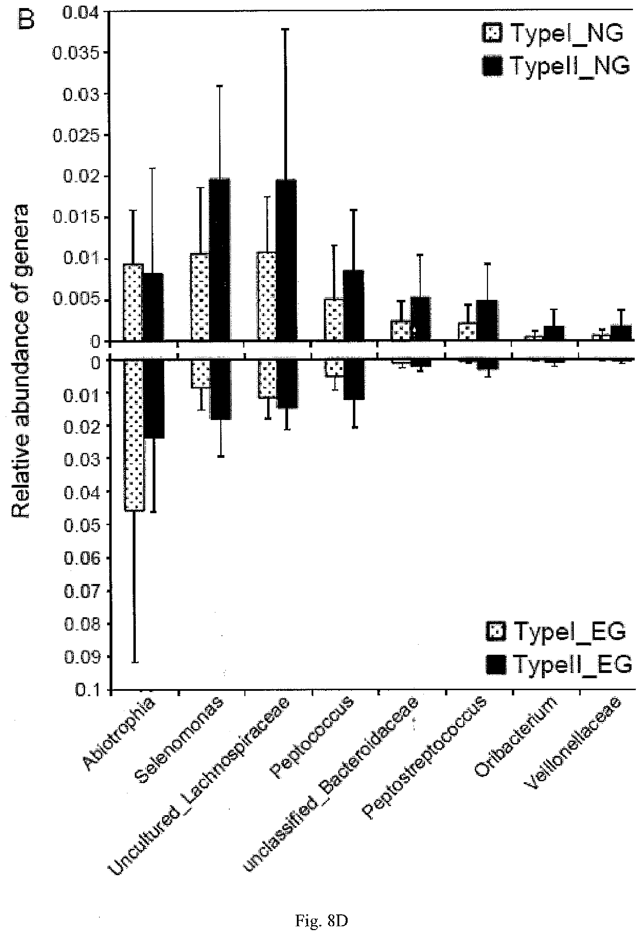

Figs. 8A, 8B, 8C, and 8D show the identification of two types of hosts with distinct sensitivity to gingivitis according to a specific embodiment of the present invention. Fig. 8A shows patterns of microbiota structural (i.e. PC1-values) change and Mazza Gingival Index change along RPM. Fig. 8B shows distribution of the 50 subjects along principal components 1 and 2 (PC1 and PC2) of the PCA, wherein the vertical dash line divides the 50 subjects into Type-I and Type-II hosts. Fig. 8C shows difference in gingivitis sensitivity between Type-I and Type-II hosts. Fig. 8D shows the abundances of 8 genus-level bacterial biomarkers that distinguish between Type-I and Type-II hosts.

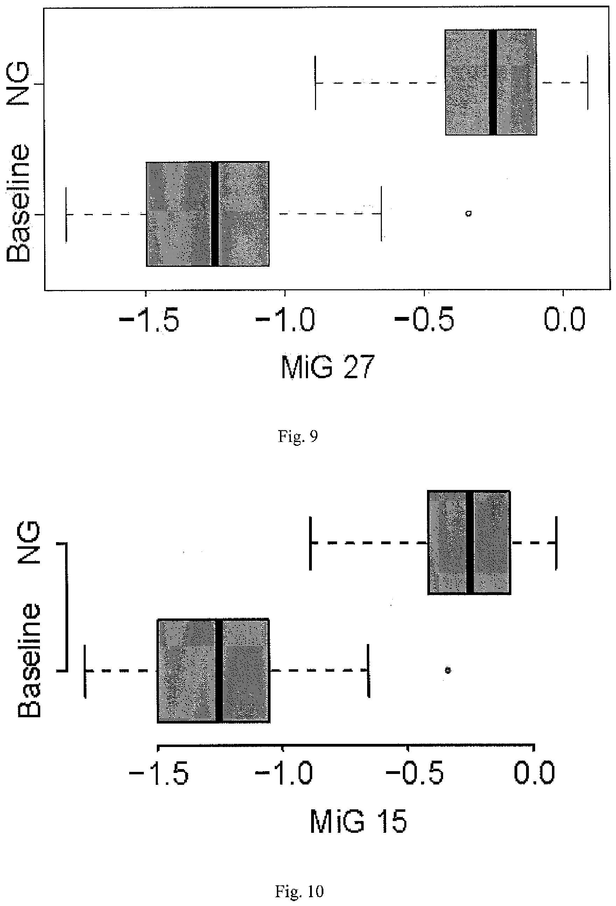

Fig. 9 shows a trial classification based on the presence of gingivitis using a microbial index of gingivitis, MiG27, which is calculated from a function based on abundances of 27 biomarkers identified according to a specific embodiment of the present invention.

Fig. 10 shows a trial classification based on the severity of gingivitis using a microbial index of gingivitis, MiG15, which is calculated from a function based on abundances of 15 biomarkers identified according to another specific embodiment of the present invention.

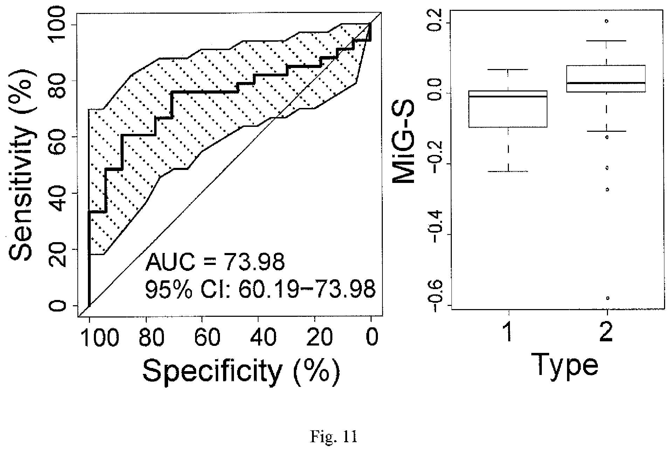

Fig. 11 shows a trial classification based on the sensitivity to gingivitis using a microbial index of gingivitis, MiG-S, which is calculated from a function based on abundances of 8 biomarkers identified according to a further specific embodiment of the present invention. The accuracy of MiG-S is measured by the area under the ROC (receiver operating characteristic) curve of plaque-microbiota-based (i.e. MiG-S-based) gingivitis-sensitive host-type classification as shown in the left diagram.

DETAILED DESCRIPTION OF THE INVENTION

## Definitions

As used herein, the term "mammal" refers to any of various warm-blooded vertebrate animals of the class Mammalia, including humans. In the context herein, the mammal can also be called "subject" or "host".

As used herein, the term "a set of mammals" means a number of mammals gathered together into a group for the purpose of study. The number in the set can be any countable number no less than 1. Depending on the purpose accuracy requirement of a specific study, the number of mammals in the set can be up to 1000, 10000 or even larger.

As used herein, the terms "microbial community", "microbiota", "microflora", "microbial flora" and "flora" are used interchangeably herein and refer to a population of diverse microorganisms that typically inhabits the mammal, specifically an organ (e.g., skin, digestive tract) or an orifice (e.g., mouth) of the mammal. The term "microorganism" means an organism of microscopic or submicroscopic size, especially a bacterium or protozoan, more preferably bacterium.

As used herein, the term "microbe-related disease" includes an illness in the mammal caused or influenced or associated by a microorganism.

As used herein, the terms "sample", "oral sample", or "biological sample" is a biological material isolated from a subject for analysis according to the present methods, such as saliva, gingival crevicular fluid (GCF), supragingival plaque, subgingival plaque, breath or exhaled air, oral lavage, tongue scrapings, swabs or biopsies from oral tissue and serum. A sample is ideally capable of containing a microbial community.

As used herein, the term "statistical significance" is a mathematical tool that is used to determine whether the outcome of an experiment is the result of a relationship between specific factor(s) or merely the result of chance. Statistical significance is used to reject or accept what is called the null hypothesis. A hypothesis is an explanation that a researcher is trying to prove. The null hypothesis typically holds that the factor(s) at which a researcher is looking have no effect on differences in the data or that there is no connection between the factors. Statistical significance is usually written, for example, as $t=0.02, p<0.05$. Here, "$t$" stands for the test score and "$p<0.05$" means that the probability of an event occurring by chance is less than 5 percent. These numbers would cause the null hypothesis to be rejected.

As used herein, with reference to a disease or condition, the term "sensitivity" and its adjective form "sensitive" can be used interchangeably with "susceptibility" and its adjective form "susceptible" to mean the likelihood of suffering from a disease or condition when exposed to a noxious stimulus or pathogen.

As used herein, the articles including "a" and "an" when used in a claim, are understood to mean one or more of what is claimed or described.

As used herein, the terms "comprise", "comprises", "comprising", "include", "includes", "including", "contain", "contains", and "containing" are meant to be non-limiting, i.e., other steps and other sections which do not affect the end of result can be added. The above terms encompass the terms "consisting of' and "consisting essentially of'.

## Retrogression-Progression Model (RPM)

The present invention is based on a retrogression-progression model (RPM) which is designed to simulate the retrogression and reoccurrence of a disease of a mammal. Oral samples are obtained at three different time points representing a naturally diseased state, a healthy state, and a reoccurring diseased state. Therefore, the RPM can be used to reveal source of the heterogeneity of microbiota both within-subject and in natural populations.

The present RPM reveals source of the heterogeneity of microbiota both within-subject at different time points as described hereinabove and between-subjects with different sensitivity to a disease. In either case, there is no clear boundary between healthy and diseased states in hosts as reflected by their microbial attributes: their distribution, as well as their retrogressive or progressive pattern, is not a discrete but rather a gradient-like process. Without wishing to be bound by any particular theory, the progression from the relatively healthy state to the diseased state is believed to be primarily driven by certain bacteria, most of which increase in abundance and some of which decrease in abundance along such progression. Therefore, the RPM can be used to simulate the retrogression and reoccurrence of a microbe-related disease, which is preferably but not necessarily an oral disease.

According to a specific embodiment, the micro-related disease is selected from the group consisting of gingivitis, periodontitis, dental caries, halitosis, oral ulcer, premature birth, low birth weight, diabetes, respiratory disease, heart disease, stroke, bacteremia, whole body health, and combinations thereof.

## Sample Collection & Storage

Depending on the specific condition, the oral sample, preferably in the form of a biofilm on the surfaces of the teeth, prostheses (when present), gums and tongue, can be selected from the group consisting of a salivary sample, a plaque sample, a tongue dorsum sample, a tongue coating sample, a mucous membrane sample, and combinations thereof. The plaque sample can be from various locations. For example, the plaque sample can be selected from the group consisting of a supragingival plaque sample, a subgingival plaque sample, a tooth plaque sample and any combination thereof. The selection of the sample may be critical to the accuracy of identifying the biomarker. For example, plaque microbiota is believed to be more sensitive to gingivitis than salivary microbiota. Therefore, in the case of gingivitis, the oral sample is preferably a plaque sample.

Treatment of the disease can be achieved by any method, only if the disease can be eliminated or reduced. For example, a therapeutically effective amount of a medicinal and/or therapeutic agent can be administrated to the subject, following a therapeutically effective regimen. In a specific embodiment, the subject mammals are supplied with a toothpaste of good quality which is capable of eliminating or reducing the disease or condition and a specific toothbrush. The subjects are asked to brush twice per day for a specific period, for example, two weeks.

Reoccurrence of the disease can be achieved by any method, only if the disease can reoccur. In most cases, the disease can be made reoccur by simply doing nothing to the disease-related parts of the subject's body. For example, in the case of gingivitis, the subject can simply following a regimen by which the subject do not have any oral hygiene practice including brushing, mouth rinsing with any products, flossing and dental prophylaxis. Optionally, a sugar, or other suitable bacterial food, can be used by the subjects at bedtime. Oral bacteria utilise the sugar overnight and generate raised levels of bacterial metabolites.

The samples, once collected, can be used in subsequent steps immediately. Alternatively, the samples can be put in a freezer for later use. In some cases, the newly collected samples are immediately deep frozen, typically below -20°C, preferably below -50°C, more preferably below -70°C, and most preferably below -90°C. The samples remain frozen until preparation for analysis.

**Microbial Community Analysis**

The mouth harbors a diverse, abundant and complex microbial community. This highly diverse microflora inhabits the various surfaces of the normal mouth. Bacteria accumulate on both the hard and soft oral tissues in biofilms. Bacterial adhesion is particularly important for oral bacteria.

Oral bacteria have evolved mechanisms to sense their environment and evade or modify the host. Bacteria occupy the ecological niche provided by both the tooth surface and gingival epithelium. Up until fairly recently, the associations between the host and oral bacteria are considered in terms of a multiplicity of single species interactions. However, it is becoming more apparent that the oral microbes comprise a complex community, and that oral health or disease depends on the interaction between the host and the microbial community as a whole. Although it is important to continue studies of the pathogenic properties of specific microbes, these are relevant only in the context of the properties of the community within which they reside. Understanding the microbial communities that drive sickness or health is a key to combating microbe-related diseases.

The potential of human microbiota for tracking and diagnosing host conditions (diseases, diets, etc) is dependent on, and limited by, the degree of heterogeneity in the link between microbiota and condition at the population level. In the gut, the variation of microbiota structure between hosts appears to dominate variation among conditions (e.g. lean or obese, or on a normal or high-fat diet). However, inventors of the present invention now surprisingly finds that the opposite appears to be true for oral microbiota, and that differences between healthy and diseased oral microbiota within a subject are larger than inter-personal differences. Although the mechanism for this difference in response sizes in microbial communities within different body habitats is unknown, the inventors' findings suggest that the oral microbiota might offer certain advantages as biomarkers for oral, and perhaps even systemic, diseases.

Therefore, according to the present invention, oral samples at three different time points, representing a naturally diseased state, a healthy state, and a reoccurring diseased state, are measured and compared to identify those microbial types whose abundances correlate with a statistical significance to a condition, wherein the condition is selected from the group consisting of: presence of a disease, severity of a disease, sensitivity to a disease, and combinations thereof.

Many techniques can be used to measure the oral sample to obtain the oral microbial community structural, functional and dynamic data. On one hand, by selecting a particular population of microorganisms, culture-based methods can be used to investigate the microbial ecology of natural and anthropogenically impacted environments. Standard culture techniques to characterize microbial ecology involve isolation and characterization of microorganisms using commercial growth media such as Luria-Bertani medium, Nutrient Agar, and Tryptic Soy Agar. The major limitation of culture-based techniques is that >99% of the microorganisms in any environment observed through a microscope are not cultivable by standard culturing techniques. On the other hand, with recent advances in genomics and sequencing technologies, a variety of culture-independent molecular methods based on direct isolation and analysis of nucleic acids, proteins, and lipids from samples have been discovered and revealed structural and functional information about microbial communities. Molecular approaches such as genetic fingerprinting, metagenomics, metaproteomics, metatranscriptomics, and proteogenomics are vital for discovering and characterizing the vast microbial diversity and understanding their interactions with biotic and abiotic environmental factors.

According to a specific embodiment, the oral sample is measured by one or more methods selected from the group consisting of 16S rRNA(RiboNucleic Acid) analysis, genetic fingerprinting, clone library method, denaturing- or temperature-gradient gel electrophoresis, random amplified polymorphic DNA(DeoxyriboNucleic Acid), DNA amplification fingerprinting, amplified ribosomal DNA restriction analysis, DNA microarrays, fluorescence *in situ* hybridization, DNA-DNA hybridization, metagenomics, metaproteomics, metatranscriptomics, proteogenomics, Luria-Bertani medium isolation technique, Nutrient Agar isolation technique, Tryptic Soy Agar isolation technique, and any combination thereof. Molecular analyses of microbial communities have revealed that the cultivable fraction represents <1% of the total number of prokaryotic species present in any given sample. Combination of the analysis methods can provide a greater comprehensive assessment of microbial diversity. Preferably, a method selecting from the group consisting of 16S rRNA analysis, metagenomics, and combination thereof is used in the present invention to measure the oral samples, obtaining abundances of one or more microbial types in the oral microbial communities. Most preferably, 16S rRNA analysis is used to study the microbial communities of the oral samples.

According to a specific embodiment, abundances of one or more microbial types in the microbial communities of the oral samples are obtained by the above one or more methods.

According to a specific embodiment, the microbial type is selected from the group consisting of taxonomic categories of a bacterium, functional categories of a microbe, and combinations thereof. More specifically and preferably, the microbial type is selected from the group consisting of a bacterial phylum, a bacterial class, a bacterial family, a bacterial order, a bacterial genus, a bacterial species, a functional gene of a microbe, a gene ortholog group of a microbe, a motif of peptide or protein of a microbe, a conserved peptide or protein domain of a microbe, a none-coding nucleotide sequence of a microbe, and combinations thereof, preferably a bacterial genus.

The present invention can be started by trying as more microbial types as possible to determine which microbial type can serve the purpose best. For example, bacterial phyla, genera and species can be respectively identified and their abundance can be respectively quantified. Significant difference in terms of the abundances of the microbial types should be able to be identified among the samples at three different time points so that the microbial community change can be identified to study the etiology of the disease. Therefore, one or more microbial types which are believed to change significantly in abundances among three different time points should be selected herein for the purpose of achieving the present invention.

According to the present invention, the abundances of the microbial types are statistically analyzed across the set of test mammals by a pair-wise comparative analysis or a multivariate analysis to identify those microbial types whose abundances correlate with a statistical significance to a condition of the set of test mammals as a first group of microbial types, wherein the condition is selected from the group consisting of: presence of the disease, severity of the disease, sensitivity to the disease, and combinations thereof.

The multivariate analysis is selected from the group consisting of principal component analysis, principal coordinate analysis, correspondence analysis, detrended correspondence analysis, cluster analysis, discriminant analysis, canonical discriminant analysis, and combinations thereof, preferably principal component analysis. Principal component analysis (PCA) is a mathematical procedure that uses an orthogonal transformation to convert a set of observations of possibly correlated variables into a set of values of linearly uncorrelated variables called principal components (PC). The number of principal components is less than or equal to the number of original variables. This transformation is defined in such a way that the first principal component (PC1) has the largest possible variance (that is, accounts for as much of the variability in the data as possible), and each succeeding component in turn has the highest variance possible under the constraint that it be orthogonal to (i.e., uncorrelated with) the preceding components. Principal components are guaranteed to be independent only if the data set is jointly normally distributed. PCA is sensitive to the relative scaling of the original variables.

In a specific embodiment, the statistical significance has a level of $p < 0.05$, preferably *$p < 0.01$,* and more preferably $p < 0.001$.

## Method of Identifying a Biomarker

One aspect of the invention provides for a method of identifying a biomarker indicative of a subject mammal's condition comprises the steps:

    a) selecting a first set of test mammals having a first disease;
    b) obtaining a first oral sample containing a first microbial community from each of the first set of test mammals having the first disease, wherein the first microbial community comprises one or more microbial types;
    c) treating each of the first set of test mammals having the first disease, who have been first oral sampled, so as to eliminate or reduce the first disease;
    d) obtaining a second oral sample containing a second microbial community from each of the first set of test mammals who have been treated, wherein the second microbial community comprises one or more microbial types;

e) making the first disease reoccur in each of the first set of test mammals who have been second oral sampled;

f) obtaining a third oral sample containing a third microbial community from each of the first set of test mammals in whom the first disease has reoccurred, wherein the third microbial community comprises one or more microbial types;

g) measuring the first, second and third oral samples to obtain abundances of the one or more microbial types in the first, second and third microbial communities, respectively;

h) statistically analyzing the obtained abundances of the one or more microbial types in the first, second and third microbial communities across the first set of test mammals to identify those microbial types whose abundances correlate with a statistical significance to a condition of the first set of test mammals as a first group of microbial types, wherein the condition is selected from the group consisting of: presence of the first disease, severity of the first disease, sensitivity to the first disease, and combinations thereof;

i) selecting one or more microbial types from the first group of microbial types as the biomarker indicative of said subject mammal's condition.

According to a specific embodiment, in step h), the obtained abundances of the one or more microbial types in the first, second and third microbial communities are statistically analyzed by a pair-wise comparative analysis comprising the steps:

1) comparing said first microbial community and said second microbial community of each of the first set of test mammals to determine change in the obtained abundances of each microbial type between said first microbial community and said second microbial community;

2) comparing the change in the obtained abundances of each microbial type from step 1) across the first set of test mammals to select those microbial types that exhibit statistically significant changes in abundances as a primary group of microbial types;

3) comparing said second microbial community and said third microbial community of each of the first set of test mammals to determine change in the obtained abundances of each microbial type between said second microbial community and said third microbial community;

4) comparing the change in the obtained abundances of each microbial type from step 3) across the first set of test mammals to select those microbial types that exhibit statistically significant changes in abundances as a secondary group of microbial types; and

5) comparing the primary group of microbial types and the secondary group of microbial types to identify those overlapped microbial types as the first group of microbial types.

According to another specific embodiment, in step h), the obtained abundances of the one or more microbial types in the first, second and third microbial communities are statistically analyzed by a multivariate analysis comprising the steps:

1) orthogonally transforming the obtained abundances of the one or more microbial types in the first, second and third microbial communities to derive a vector accounting for the largest variance among the obtained abundances; and

2) identifying those microbial types with the obtained abundances that exhibit statistically significant correlations to the derived vector as the first group of microbial types.

Interestingly, the present inventors' study also unravels a microbial link to the heterogeneity of disease outcome in mammal population, which makes it possible to separate the mammal population into disease-sensitive mammals and less disease-sensitive mammals based on the microbial ecology. Without being bound to any theory, it is found that disease-sensitive mammals are characterized by more acute changes in microbial community structure from a healthy state to a diseased state than less disease-sensitive mammals.

Therefore, according to another specific embodiment, in step h), the obtained abundances of the one or more microbial types in the first, second and third microbial communities are statistically analyzed by a multivariate analysis comprising the steps:

1) orthogonally transforming the obtained abundances of the one or more microbial types in the first, second and third microbial communities to derive a vector accounting for the largest variance among the obtained abundances;

2) projecting the obtained abundances of the one or more microbial types in each of the first, second and third microbial communities of each of the first set of test mammals on the derived vector to obtain a projection value for each of the first, second and third microbial communities of each of the first set of test mammals;

3) calculating a change rate of the projection values across the first, second and third microbial communities for each of the first set of test mammals;

4) classifying the first set of test mammals, based on the calculated change rates, into a first subset of test mammals and a second subset of test mammals, wherein the first subset of test mammals exhibit greater change rates than the second subset of test mammals; and

5) comparing the first, second and third microbial communities of the first subset of test mammals with the first, second and third microbial communities of the second subset of test mammals, respectively, to identify those microbial types whose abundances in each of the first, second and third microbial communities are statistically significantly different between the first subset of test mammals and the second subset of test mammals, as the first group of microbial types.

According to a further embodiment, the present method further comprises the steps:

1) selecting a second set of test mammals having a second disease;

2) repeating steps b) to h) to identify a second group of microbial types;

3) comparing the first group of microbial types and the second group of microbial types to identify those overlapped microbial types as a subgroup of microbial types; and

4) selecting one or more microbial types from said subgroup of microbial types as the biomarker indicative of said subject mammal's condition, wherein the condition is selected from the group consisting of: presence of the first disease and the second disease, severity of the first disease and the second disease, sensitivity to the first disease and the second disease, and combinations thereof.

**Computer-aided System for Identifying a Biomarker**

According to the present invention, a computer-aided system helpful in practicing the method of the present invention is provided. The present computer-aided system of identifying a biomarker indicative of a subject mammal's condition comprises:

a) a sampling section for sampling:

1) a first oral sample containing a first microbial community from each of a set of test mammals having a disease, wherein the first microbial community comprises one or more microbial types,

2) a second oral sample containing a second microbial community from each of the set of test mammals who have been treated to eliminate or reduce the disease, wherein the second microbial community comprises one or more microbial types, and

3) a third oral sample containing a third microbial community from each of the set of test mammals in whom the disease has reoccurred, wherein the third microbial community comprises one or more microbial types;

b) a measuring section in communication with the sampling section, wherein the measuring section is configured for measuring the first, second and third oral samples to obtain abundances of the one or more microbial types in the first, second and third microbial communities, respectively; and

c) a computing section in communication with the measuring section, wherein the computing section is configured for receiving and statistically analyzing the obtained abundances of the one or more microbial types in the first, second and third microbial communities across the set of test mammals to identify those microbial types whose abundances correlate with a statistical significance to a condition of the set of test mammals as the biomarker indicative of said subject mammal's condition, wherein the condition is selected from the group consisting of: presence of the disease, severity of the disease, sensitivity to the disease, and combinations thereof.

The sampling section may comprise one or more devices in the form selected from the group consisting of a spoon, a cotton swab, a blade, a brush, a probe, and any combination thereof. In a specific embodiment, the sampling section comprises a sterile cotton swab, and the sampling is accomplished by gently rubbing exposed tooth surfaces with the sterile cotton swab.

In a specific embodiment, the present system can comprise a sample storage section for storing samples. If the samples collected from the sampling section are not to be used immediately, it is recommended to store them in the sample storage section. In a further specific embodiment, the sample storage has a temperature adjustment unit which can provide the sample storage section with a wide range of storing temperature, preferably below 30°C and more preferably below 0°C. In a preferred embodiment, the sample storage section provides a storing temperature of below -20°C, preferably below -50°C, more preferably below -70°C, and most preferably below -90°C.

The measuring section may comprise a sub-section performing one or more methods selected from the group consisting of 16S rRNA analysis, genetic fingerprinting, clone library method, denaturing- or temperature-gradient gel electrophoresis, random amplified polymorphic DNA, DNA amplification fingerprinting, amplified ribosomal DNA restriction analysis, DNA microarrays, fluorescence *in situ* hybridization, DNA-DNA hybridization, metagenomics, metaproteomics, metatranscriptomics, proteogenomics, Luria-Bertani medium isolation technique, Nutrient Agar isolation technique, Tryptic Soy Agar isolation technique, and any combination thereof. The microbial community structural or functional data may vary with the specific method embodied in the measuring section.

The computing section can be in any form. For example, it can be a personal computer or a portable device which comprises a computing program. According to a specific embodiment, the computing section comprises:

1) an input module in communication with the measuring section, wherein the input module is for inputting the obtained abundances of the one or more microbial types in the first, second and third microbial communities;
2) a data processing module in communication with the input module, wherein the data processing module is configured for statistically analyzing the inputted abundances of the one or more microbial types in the first, second and third microbial communities across the set of test mammals to identify those microbial types whose abundances correlate with a statistical significance to the condition; and
3) an output module in communication with the data processing module, wherein the output module is for displaying those identified microbial types as the biomarker indicative of said subject mammal's condition.

According to a further specific embodiment, the data processing module comprises a program for conducting a pair-wise comparative analysis or a multivariate analysis upon the inputted abundances of the one or more microbial types in the first, second and third microbial communities.

According to a further specific embodiment, the data processing module comprises a program for conducting a pair-wise comparative analysis upon the inputted abundances of the one or more microbial types in the first, second and third microbial communities, the program comprising instructions for:

1) comparing said first microbial community and said second microbial community of each of the set of test mammals to determine change in the inputted abundances of each microbial type between said first microbial community and said second microbial community;
2) comparing the change in the inputted abundances of each microbial type from step 1) across the set of test mammals to select those microbial types that exhibit statistically significant changes in abundances as a primary group of microbial types;
3) comparing said second microbial community and said third microbial community of each of the set of test mammals to determine change in the inputted abundances of each microbial type between said second microbial community and said third microbial community;
4) comparing the change in the inputted abundances of each microbial type from step 3) across the set of test mammals to select those microbial types that exhibit statistically significant changes in abundances as a secondary group of microbial types; and
5) comparing the primary group of microbial types and the secondary group of microbial types to identify those overlapped microbial types.

According to a further specific embodiment, the data processing module comprises a program for conducting a multivariate analysis upon the inputted abundances of the one or more microbial types in the first, second and third microbial communities, the program comprising instructions for:

1) orthogonally transforming the inputted abundances of the one or more microbial types in the first, second and third microbial communities to derive a vector accounting for the largest variance among the inputted abundances; and
2) identifying those microbial types with the inputted abundances that exhibit statistically significant correlations to the derived vector.

According to a further specific embodiment, the data processing module comprises a program for conducting a multivariate analysis upon the inputted abundances of the one or more microbial types in the first, second and third microbial communities, the program comprising instructions for:

1) orthogonally transforming the inputted abundances of the one or more microbial types in the first, second and third microbial communities to derive a vector accounting for the largest variance among the inputted abundances;
2) projecting the inputted abundance of the one or more microbial types in each of the first, second and third microbial communities of each of the set of test mammals on the derived vector to obtain a projection value for each of the first, second and third microbial communities of each of the set of test mammals;

3) calculating a change rate of the projection values across the first, second and third microbial communities for each of the first set of test mammals;

4) classifying the first set of test mammals, based on the calculated change rates, into a first subset of test mammals and a second subset of test mammals, wherein the first subset of test mammals exhibit greater change rates than the second subset of test mammals; and

5) comparing the first, second and third microbial communities of the first subset of test mammals with the first, second and third microbial communities of the second subset of test mammals, respectively, to identify those microbial types whose abundances in each of the first, second and third microbial communities are statistically significantly different between the first subset of test mammals and the second subset of test mammals.

In a specific embodiment, the sampling section, the measuring section and the computing section, alone or in any combination, can be implemented as a computer program product comprising computer executable instructions embodied in a computer readable medium. Exemplary computer readable media include chip memory devices, disk memory devices, flash memory devices, programmable logic devices, application specific integrated circuits, downloadable electrical signals, and the like. In addition, a computer program product suitable for the present invention may be located on a single device or computing platform or may be distributed across multiple devices or computing platforms.

As necessary, one or more of the sections as stated above can be compacted into a large-size apparatus or a small-size portable device.

EXAMPLES

The examples herein are meant to exemplify the present invention but are not used to limit or otherwise define the scope of the present invention.

**List of Acronyms:**

NG: naturally occurring gingivitis
EG: experimental gingivitis
MGI: Modified/Mazza Gingival Index
BOP: Bleeding on Probing
MiGs: Microbial indices of Gingivitis
RPM: retrogression-progression model
PAM: Partitioning Around Medoids (clustering algorithm)
PCA: Principal Component Analysis
PCoA: Principal Coordinates Analysis
FDR: False Discovery Rate
COG: Clustering of Orthologous Groups
MiG: microbial index of gingivitis
MiG-S: microbial index of gingivitis sensitivity
ROC: receiver operating characteristic
CI: confidence interval
KO: KEGG Ortholog

**RPM** *design* Fig. 1A illustrates a design of longitudinal study simulating gingivitis development in human population. The experimental model of gingivitis is established as a non-invasive model in humans for understanding pathogenesis of gingivitis. Experiments are conducted at Procter & Gamble (Beijing) Technology Co., Ltd. Oral Care Department, with approval from the P&G Beijing Technical Center (China) Institutional Review Board and in accordance with the World Medical Association Declaration of Helsinki (1996 amendment). ICH Guidelines for Good Clinical Practice (GCP) are followed. Ninety-one subjects are recruited from the Beijing area. Voluntary informed consent is provided.

Individuals meeting the following criteria are included: be at least 18 years of age; possess a minimum of 12 natural anterior teeth; have at least 5 bleeding sites as measured by Mazza Gingival Index (MGI) at initial visit (Day -21); have gingivitis but not periodontitis; be in good general health as determined by the Investigator/designee based on a review of the medical history/update for participation in the study. Exclusion criteria for individuals includes: severe periodontal disease, as characterized by purulent exudates, generalized mobility, and/or severe recession; any condition which requires antibiotic premedication for the administration of a dental prophylaxis; self-reported pregnancy or intent to become pregnant during the course of the study and nursing females; atypical discoloration or pigmentation in the gingival tissue; fixed facial orthodontic appliances; atypical discoloration or pigmentation in the gingival tissue; use of antibiotics any time during the study; any diseases or conditions that could be expected to interfere with the subject safely completing the study. Clinical parameters for each subject are measured per week across the whole study. Individuals that fell into the exclusion criteria at any time point are excluded from study participation.

The RPM includes three phases.

Phase I, Oral Hygiene Phase (Day -21 to Day 0): Gingivitis examinations using Mazza gingival index are conducted at -21, -14, -7 and 0 days. After receiving a dental prophylaxis (super and sub gingival prophylaxis) and tooth polishing, each subject is instructed to return to the site twice daily at which time they brush under supervision using Mei Li Liang Jie manual toothbrush (Crest, Made in China) for three minutes with a currently marketed anti-cavity dentifrice without any marked anti-microbial actives and then use the floss to clean the dental interproximal area. This brushing regimen is followed for the next 21 days while recording MGI for each subject each visit. During the Oral Hygiene Phase, subjects receive up to three dental prophylaxes if the subjects bleeding sites are more than 1.

Phase II, Experimental Gingivitis Phase (Day 0 to Day 21): During this phase, subjects do not have any oral hygiene practice including brushing, mouth rinsing with any products, flossing and dental prophylaxis. Subjects also receive a gingivitis exam at days 7, 14 and 21 of the Experimental Gingivitis Phase.

Phase III, Recovery Phase: Subjects are instructed to return to the site twice daily at which time they brush under supervision using products and techniques in Phase I. Subjects receive a dental prophylaxis during the Recovery Phase and the subjects also receive gingivitis exam, inclusive of measured bleeding sites, to document and confirm that they have been returned to equivalent or preferably better health than when they enter the study. If needed, subjects receive an additional prophylaxis and are monitored until deemed healthy.

Gingivitis is assessed using Bleeding on Probing (BOP) and Mazza Gingival Index (MGI) as clinical measurements. BOP frequency and mean MGI are recorded for each subject. MGI measures both the signs of inflammation and the degree of the severity of bleeding. Specifically, probing is performed by a dentist on the mesiobuccal and the distolingual of each tooth, for a maximum of 56 sites. Scores range from 0 to 5, with 0 assigned for normal appearing and healthy gingival up to a score of 5 for spontaneous bleeding (without provocation). MGI of all subjects are measured by the same well-trained dentist to reduce technical variation.

Fig. 1B shows changes of the above clinical parameters for 50 subjects cross the study. In Fig. 1B, boxes represent the interquartile range (IQR) and the lines inside represent the median. Whiskers denote the lowest and highest values within 1.5x IQR. At -21 day, all subjects exhibit a certain level of gingival inflammation that represents the state of naturally occurring gingivitis ("NG") with BOP ranging from 5 to 27 and average MGI from 1.18 to 2.24. These subjects then undergo rigorous oral hygiene practice for three weeks, which results in a greatly reduced BOP and MGI (Median BOP and MGI are 1.00 and 1.02 respectively) at 0 day ("Baseline") that represents a healthy gum state. Then the hosts further undergo an oral hygiene program for gingivitis induction for three weeks that results in significantly increased BOP (median 23) and MGI (median 2.11) representing the state of experimental gingivitis ("EG").

*Supragingival plaque sampling* Supragingival plaque samples from each subject are collected at Day -21, Day 0 and Day 21 following the procedures below. Subjects do not have oral hygiene practice including tooth brushing, flossing, mouth rinsing before sampling. Samples are collected after 2 hours food or drink (except water) intake. After MGI examination, each subject rinses their mouth with 50ml sterilized water. After MGI examination 15 minutes, plaque along the gumline within 2 mm depth are collected with Gracey curette by qualified dentists. For each subject, plaque samples are collected for all teeth in two different quadrants (1 and 3 or 2 and 4) and pooled together in one tube. Plaques on the Gracey curette are collected via swabbing with a sterilized cotton swab. The tips of swab are put into 0.6 ml TE20 buffer (20 mM Tris-HCl PH 8.0, 2 mM EDTA (ethylenediaminetetraacetic acid)). Before isolating DNA, all samples are stored under -70°C.

*Plaque DNA extraction protocol* Total DNA is extracted from Human Dental plaque following Dr. Larry Fernery's protocol with minor modifications (Ravel J, et al. (2011) Vaginal microbiome of reproductive-age women. Proc. Natl. Acad. Sci. U. S. A. 108:4680-4687). In general, frozen samples are thawed on ice before DNA isolation experiment. The original sample (250 $\mu$l) is transferred into a clean Bead-Beating-Tube (2ml Eppendorf tube). Sample suspensions are kept on ice while a Lytic-Enzyme Cocktail is prepared. Freshly prepared Lytic-Enzyme-Cocktail Master-Mix (100ul; containing 50 $\mu$l Lysozyme~500KU=10mg/ml, 6 $\mu$l Mutanolysin, 25 KU/ml, 3 $\mu$l Lysostaphin, 4000 U/ml in 20 mM sodium acetate and 41 $\mu$l TE buffer) is added to all samples and incubated at 37°C for 45 min. To the lysate mix 750 mg cleaned and dry 0.1mm diameter Zirconia-Silica-Beads is added. Samples are subjected to bead beating for 2 minutes at room temperature in a Qiagen TissueLyser LT (36 oscillations/ second). One hundred and eighty $\mu$l of the crude lysate are transferred into a new tube and DNA isolated by Qiacube using DNeasy® Blood & Tissue Mini Kits.

*Bacterial 16S rRNA gene amplicon sequencing* 150 plaque samples are obtained and analyzed from 50 individuals each of whom provides samples at the three timepoints of NG (Day -21), Baseline (Day 0) and EG (Day +21). Barcoded 16S rDNA amplicon sequencing using 454 Titanium yields a total of 3,181,659 raw reads, resulting in totally 1,093,922 processed reads (i.e., reads after quality assessment and control measures). The number of processed reads per sample ranges from 437 to 28, 456, with an average 7293 reads per sample. All sequences are deposited at Sequence Read Archive under Accession ID SRA058763.

***Comparing the phylogenetic structures of plaque microbiota*** PCA analysis is first performed in R using the ade4 package (Dray S & Dufour AB (2007) The ade4 package: Implementing the duality diagram for ecologists. Journal of Statistical Software 22(4):1-20) to visualize the difference of microbial community structure among different time points. Procrustes analysis attempts to stretch and rotate the points in one matrix, such as points obtained by PCA, to be as close as possible to points in the other matrix, thus preserving the relative distances between points within each matrix. Simple Procruste rotation in R using the ade4 package between two subsets of transformed data (i.e. data matrix of first-four principal components of NG-baseline, EG-baseline and NG-EG) is performed to test the degree of difference among different time points for the microbiota of the cohort.

Principal coordinates analysis (PCoA) is also performed to confirm the difference of microbiota structure between populations of gingivitis and health. In each sample, representative sequences from each OTU (operational taxonomic unit) are chosen by selecting the longest sequence based on UCLUST (Edgar RC (2010). Search and clustering orders of magnitude faster than BLAST. Bioinformatics 26(19):2460-2461). Each sequence is assigned to its closest relative in the phylogeny in CORE (Griffen AL, et al. (2011) CORE: a phylogenetically-curated 16S rDNA database of the core oral microbiome. PLoS One 6(4):e19051) using BLAST'S megablast. The resulted sample ID (identification) mapping file and category mapping file are used as inputs to FastUniFrac (Hamady M, Lozupone C, & Knight R (2010) Fast UniFrac: facilitating high-throughput phylogenetic analyses of microbial communities including analysis of pyrosequencing and PhyloChip data. ISME J 4(1):17-27), which allows pairwise comparisons of inter-community distances based on the fraction of evolutionary history that separates the organisms. These distances are then clustered to reduce dimensionality using PCoA, where the principal coordinates (PC) describe in descending order the degree of variation that each of the axes in the new space explains. In addition, ThetaYC-based community structure comparisons are performed using MOTHUR (Schloss PD, Gevers D, & Westcott SL (2011) Reducing the effects of PCR (Polymerase Chain Reaction) amplification and sequencing artifacts on 16S rRNA-based studies. PLoS One 6(12):e27310). ThetaYC measures the structural dissimilarity between two communities. A matrix of pairwise thetaYC-based distances among all samples is created for clustering and PCoA analysis.

***Statistical analysis*** To test the structural heterogeneity of microbiota, clustering among the plaque microbiota is performed by partitioning around medoids (PAM) using Jensen-Shannon divergence (JSD) of the normalized genus (or OTU) abundance. The optimal number of clusters is chosen based on the maximum of the silhouette index.

PCA analysis is then performed in R using the ade4 package to visualize the clustering based on PAM. Prior to the analysis, the data are sample-size normalized and very low abundant genera are removed (if their average abundance across all samples is below 0.1%) to decrease noise. Bacterial genera that exhibit the highest correlation to PC1 are identified and highlighted.

The weighted correlation network analysis (WGCNA) is used to study microbial associations and interaction. This method is applied to construct bacterial interaction networks in plaque. In these networks, a node corresponds to the microbial abundance profile of a given microbe. Nodes are connected if they have a significant pairwise correlation across the environmental perturbations. Pairwise Pearson correlations between all genera across all subjects are first calculated. The soft thresholding power of the correlation is then identified to construct a robust network following the criterion of approximate scale-free topology. Topological overlap of genera is calculated to reflect their relative interconnectedness. Finally, data is exported and visualized via Cytoscape (http://www.cytoscape.org). The power of the pairwise Pearson correlation is β = 3 at EG, with scale free topology fit index = 0.7. Oral bacteria (genus level) that have average relative abundance above 0.1% and strength of connection between two bacteria > 0.05 are plotted in the networks.

To evaluate the effect of plaque microbiota on gingivitis, the present inventors define and compute the microbial index of gingivitis for each individual on the basis of the selected phylogenetic markers (biomarkers) by either paired *t*-test or spearman correlation method. For each individual sample, the microbial gingivitis index denoted by *f* (*Ai, Aj*) is computed by the formula below:

$$f(Ai, Aj) = \mathrm{b}\left(\frac{\sum_{i \in N} Ai}{N} - \frac{\sum_{j \in M} Aj}{M}\right)$$

where *N* is a total number of the gingivitis-enriched markers in these selected phylogenetic markers, M is a total number of the health-enriched markers in these selected phylogenetic markers, *Ai* is an abundance of each gingivitis-enriched marker *i, Aj* is an abundance of each health-enriched marker *j,* $\sum_{i \in N} Ai$ is a sum *of Ai* over all gingivitis-enriched markers *i,* $\sum_{j \in M} Aj$ is a sum *of Aj* over all health-enriched markers *j,* and b is a constant which can be 10 or any other number.

***Plaque metagenome sequencing*** For 18 of the plaques, metagenomic DNA are separately extracted and sequenced. The samples are at both Baseline and EG and from nine of the subjects, including five subjects from Gingivitis-cluster I and four from Gingivitis-cluster II. The paired-end sequencing libraries are prepared under the NEXTflex™ technology (BIO Scientific Corp., USA). Metagenomic DNA is first fragmented with liquid nitrogen. Sequencing adaptors that include the index sequences are then ligated on the size-selected fragments. Ten cycles of PCR are introduced to enrich the properly ligated fragments. The enriched products are then sequenced on HiSeq (Illumina, USA) with 2 x150bp read length. These reads are subjected to quality filtering, and then human reads identified and separately archived. All sequences are deposited at Sequence Read Archive under Accession ID SRA058763.

*Functional Classification of genes* To probe the encoded functions, the microbial reads are assembled into contigs using IDBA (http://i.cs.hku.hk/~alse/hkubrg/projects/idba/) with default parameters. The assembled contigs are then submitted to MetaGeneMark for gene calling using default parameters. The gene fragments are then functionally assigned to the COG database using BLAST and a perl script. More than 60% of the genes are annotated by COG. PCA of functional gene profiles based on COG assignment are generated by R (2.15.1).

Fig. 2 shows a flow chart summarizing the study pipeline as discussed hereinabove.

## Results

For each of the 150 plaque microbiota, bacterial phyla, genera and species are identified and their relative abundances quantified via taxonomic assignment against reference databases (CORE (Griffen AL, et al. (2011) CORE: a phylogenetically-curated 16S rDNA database of the core oral microbiome. PLoS One 6(4):e19051)).

*An experimentally tractable model of gingivitis retrogression and progression* As shown in Fig. 1B, at the population level, MGI ($p$<0.001) and BOP (p=0.026) are significantly higher at EG (mean BOP 26.00±9.59 and MGI 2.12±0.48) than at NG (mean BOP 13.5±5.12 and MGI 1.61±0.24) based on paired $t$-tests. Furthermore, for individual subjects, clinical parameters between NG and EG are significantly correlated, such as BOP (Pearson Correlation: $r$=0.31, $p$=0.03) and mean MGI (Pearson Correlation: $r$=0.35, $p$=0.01).

*Structural and functional features of gingivitis-associated microbiota* To identify structural features of microbiota associated with gingivitis, all 150 healthy and diseased microbiota are clustered via PCA based on the relative abundance of genera-level taxa, and distinction in organismal structure between healthy (Baseline, triangles) and gingivitis-associated plaque microbiota (NG, diamonds; EG, dots) is observed (see Fig. 4A). The healthy and diseased microbiota are largely concentrated along the boundary of Baseline and NG/EG, suggesting a connection between microbiota structure and disease state. The higher MGI at EG within-subject structures between NG and EG are largely consistent, suggesting that microbial community perturbations associated with gingivitis recur the same way in the same subjects. These results are also supported by PCoA based on UniFrac and ThetaYC distances (see Figs. 5A and 5B). Thus a microbiota-disease link within each subject might be present.

The present inventors examin the microbiota-disease link by correlating the relative abundance of all bacteria taxa with host-states.

At the phylum level, nearly all sequences are from 13 bacterial phyla, including six predominant bacterial phyla commonly encountered in the oral cavity: *Firmicutes, Proteobacteria, Bacteroidetes, Actinobacteria, Fusobacteria* and *TM7* (each with average relative abundance > 1% at least one timepoint). Between the gingivitis states (NG and EG) and the healthy gingival state (Baseline), significant difference (p < 0.01; paired $t$-test) are found in five predominate phyla: *Actinobacteria, Firmicutes, TM7, Bacteroidetes* and *Fusobacteria.* A temporal shift of community-structure along the NG-Baseline-EG progression is apparent, characterized by the elevated relative abundance of *Actinobacteria* and *Firmicutes* at Baseline, and that of *TM7, Bacteroidetes* and *Fusobacteria* at NG and EG.

At the genus level, 27 bacterial genera (each with average relative abundance >0.1% at least one time point) are differentially distributed ($p$<0.05, paired $t$-test; FDR (false discovery rate) $q$<0.2) between Baseline and gingivitis (both NG and EG). Among them, five *(Streptococcus, Rothia, Actinomyces, Haemophilus* and *Lautropia)* show elevated abundance at Baseline, while 22 *(Leptotrichia, Prevotella, Fusobacterium,* TM7, *Porphyromonas, Tannerella, Selenomonas, Uncultured_Lachnospiraceae, unclassified_Comamonadaceae, Peptococcus, Aggregatibacter, Catonella, Treponema,* SRI, *Campylobacter, Eubacterium, Peptostreptococcus, unclassified_Bacteroidaceae, Solobacterium, Johnsonella, Oribacterium,* and *unclassified_Veillonellaceae)* are enriched in both NG and EG. Fig. 3 shows these 27 genus-level bacterial biomarkers that are believed to denote gum health and Gingivitis (for both naturally occurring gingivitis and experimental gingivitis). Relative abundance of identified oral bacteria in microbial community at different stages is also displayed. These bacterial taxa can potentially serve as disease markers.

The current clinical practice of separating hosts into diseased and healthy groups is based on the arbitrary MGI-cut-off of 1.10~1.12. However, such a bimodal definition of disease and health is contrary to the observed characteristics of hosts and microbiota. To visualize distribution of mean MGI value among the samples, the data points of PCA are scaled by the mean MGI value for each sample in Fig. 4B. Mean MGI and PC1 values show significant correlation ($p$<0.05). Therefore, in fact, the distribution of clinical parameters (e.g. MGI) both within individual hosts and in human populations is continuous. Moreover, PCA analysis suggests that the transition of the microbiota between NG, Baseline and EG is not a discrete process, but rather gradient-like (see Fig. 4A). Therefore a new clinical model is required that considers the distribution of both disease phenotype and microbiota structure along a gradient, which should be useful for providing a more objective measure of disease states and allowing more appropriate statistical tests of links between the microbiota and disease.

The projected coordinate of a given microbiota on the PC1 appears to capture the gradient-like heterogeneity and development of microbiota structure along disease retrogression and progression, as changes in PC1 within subjects and across cohorts are largely consistent with the structural segregation between healthy and diseased microbiota (see Fig. 4B). Moreover, the relative order of microbiota along PC1 defined using all 150 samples is similar to those defined using healthy-only, NG-only or EG-only microbiota alone (Spearman correlation; All vs Healthy-only: $rho$=0.95, $p$<0.001; All vs NG: $rho$= 0.97, $p$<0.001; All vs EG: $rho$= 0.97, $p$<0.001). Therefore PC1 appears to be the primary descriptor and thus a good proxy for quantitatively measuring the development of the microbiota in both RPM-segments (e.g. NG-to-Baseline and Baseline-to-EG).

For the 50 hosts along RPM, 15 bacterial genera are found to be the drivers of microbiota heterogeneity along PC1, as their gradients in abundance are significantly correlated with the coordinates of their corresponding samples on PC1 (Spearman $rho$>0.7, FDR q<0.2), as shown in Table 1 below.

Table 1. Oral bacterial that shows significant correlation with PC1

| Genus | Rho value |
|---|---|
| *Rothia* | -0.76 |
| *Haemophilus* | -0.7 |
| *Prevotella* | 0.85 |
| *Leptotrichia* | 0.81 |
| *Fusobacterium* | 0.71 |
| *Selenomonas* | 0.85 |
| uncultured *Lachnospiraceae* | 0.83 |
| TM7 | 0.81 |
| *Tannerella* | 0.74 |
| *Peptococcus* | 0.82 |
| *Peptostreptococcus* | 0.73 |
| *Catonella* | 0.73 |
| *Treponema* | 0.82 |
| *Solobacterium* | 0.72 |
| unclassified Bacteroidaceae | 0.72 |

These drivers include *Rothia, Haemophilus, Prevotella, Leptotrichia, Fusobacterium, Selenomonas,* uncultured *Lachnospiraceae,* TM7, *Tannerella, Peptococcus, Peptostreptococcus, Catonella, Treponema, Solobacterium* and unclassified Bacteroidaceae. Two of the 15 genera, *Rothia* and *Haemophilus,* decrease in relative abundance along PC1 ("negative drivers"), while the other 13 increase along PC1 ("positive drivers"). To understand their ecological roles in gingivitis development at host population, bacteria correlation networks at NG, Baseline and EG respectively are created using the relative abundance of bacterial genera in the 50 hosts. Interestingly, in the EG network, the drivers *Prevotella, Selenomonas, uncultured_Lachnospiraceae, Catonella, Peptostreptococcus, Treponema, Haemophilus* and *Leptotrichia* are the top eight most connected nodes, suggesting they are the major hubs of bacterial interactions underlying gingivitis. Interactions among the 15 bacterial PCI-driving genera are similar at the three different time points: in each of the NG, Baseline and EG networks, the 13 positive drivers interact positively with each other and interacted negatively with the two negative drivers. Interestingly, the network is more connected at EG and Baseline than at NG among the 15 PC1-drivers (see Fig. 6A, 6B and 6C), with merely 24 connections (14 nodes) in the NG network, despite the presence of 65 connections (14 nodes) in the Baseline network and 54 connections (15 nodes) in the EG network. In Fig. 6A, 6B and 6C, size of nodes is proportional to the relative abundance of the taxa. Those taxa whose relative abundance are significantly correlated are connected by 'links' (solid: positive correlation; dotted: negative correlation). Thus, uncontrolled environmental factors at NG might have concealed certain inter-microbe links, which underscores the value of experimental disease models such as RPM.

To test the functional features of gingivitis microbiota, the genomic DNA from 18 of the plaques (from nine of the subjects, each of whom sampled at both Baseline and EG) are shotgun-sequenced respectively at a depth of averagely 3.94 Gb per sample (see Table 2).

Table 2. Features of metagenome shotgun sequences produced for the 150 plaque microbiota.

| Host id | Age | Sex | Baseline | | | | | EG (experimentally induced gingivitis) | | | | |
|---------|-----|-----|----------|---|---|---|---|---|---|---|---|---|
| | | | Sample ID | Mean MGI | Bleeding sites number | Metagenome sample size (Gb) | Metagenome Reads | Sample ID | Mean MGI | Bleeding sites number | Metagenome sample size (Gb) | Metagenome Reads |
| 9066 | 32 | F | 9066B | 1.00 | 0 | 3.92 | 26110020 | 9066E | 1.93 | 26 | 4.05 | 26999772 |
| 9174 | 36 | F | 9174B | 1.05 | 2 | 3.75 | 25005760 | 9174E | 2.63 | 39 | 4.14 | 27567214 |
| 9183 | 26 | M | 9183B | 1.02 | 1 | 4.15 | 27644386 | 9183E | 1.77 | 19 | 3.91 | 26036894 |
| 9439 | 27 | F | 9439B | 1.00 | 0 | 3.73 | 24860734 | 9439E | 1.68 | 19 | 4.08 | 27193368 |
| 9445 | 27 | M | 9445B | 1.05 | 2 | 3.25 | 21661610 | 9445E | 1.98 | 26 | 4.08 | 27186152 |
| 9147 | 28 | M | 9147B | 1.04 | 2 | 3.99 | 26585026 | 9147E | 2.34 | 29 | 3.96 | 26410660 |
| 9148 | 41 | F | 9148B | 1.07 | 2 | 4.02 | 26815920 | 9148E | 3.13 | 41 | 3.27 | 21798906 |
| 9307 | 34 | F | 9307B | 1.04 | 2 | 4.14 | 27567214 | 9307E | 2.33 | 32 | 4.21 | 28041294 |
| 9325 | 32 | F | 9325B | 1.00 | 0 | 4.14 | 27590228 | 9325E | 2.52 | 36 | 4.09 | 27281026 |

These nine subjects are picked to maximize the phylogenetic diversity of microbiota sampled. Functional genes encoded in the microbiota are analyzed based on Clusters of Orthologous Groups (COG) Database and compared based on relative abundance of the assigned orthologous groups (OG). Interestingly, Procrustes analysis indicates that, among the 18 samples, the agreement between phylogenetic and functional measurements (COG) is excellent ($p<0.001$ by 10000 Monte Carlo label permutations). As shown in Fig. 7A, disease state significantly affects the microbial community function. The effects of disease state on the microbial community functions appear to be well separated by the first axis. Furthermore, as shown in Fig. 7B, clustering of the 18 microbiota based on encoded functional genes is nearly identical to that based on organismal structure, suggesting gingivitis microbiota are distinct from healthy in functional gene structure. In Fig. 7B, the fit of each Procrustes transformation over the first four dimensions is reported as the p value by 10000 Monte Carlo label permutations. In total, 1205 OGs involving 24 functional categories (out of 4873 OGs in 25 categories) are either positively or negatively gingivitis-associated ($p<0.01$). For example, in Functional Category N (cell motility), 33 OGs mostly relate to flagellar biosynthesis pathways are enriched in gingivitis, while merely three OGs (all related to pilus assembly protein) are enriched in healthy hosts. Fig. 7C shows the 33 gingivitis-enriched OGs that encode components of the flagellar biosynthesis pathway. The schematic is adapted from Kyoto Encyclopedia of Genes and Genomes (KEGG), with gene names of the corresponding KO (KEGG Ortholog) highlighted. On the other hand, in Functional Category P (inorganic ion transport and metabolism), 32 OGs are enriched in healthy microbiota while only 19 are depleted. Thus gingivitis microbiota is distinct from healthy ones in both structure and function.

*Link between PC1 and disease phenotype* The classification of healthy and diseased microbiota under the 16S-taxonomy-based PCA is identical to that under the functional-gene-based PCA, suggesting that the value of each sample along the PC1 axis is a useful descriptor for both structural and functional features of gingivitis microbiota.

The PC1-value appears to harbor clinically useful information. Tables 3 and 4 show correlation between alteration of microbiota structure (ΔPC1) and change of MGI (ΔMGI). Table 2 refers to the 50-host cohort at the three phases of NG-Baseline, Baseline-EG and NG-EG. Table 3 refers to the additional 41-host cohort at the single stage of NG-Baseline. Only the rho values with its corresponding $p<0.05$ (i.e. significant correlation) are shown.

Table 3

| Spear man correlati on (rho) | | NG to Baseline* | | | | Baseline to EG | | | | NG to EG | | | |
|---|---|---|---|---|---|---|---|---|---|---|---|---|---|
| | | NG.PC1 | ΔPC1 | NG.MGI | ΔMGI | B.PC1 | ΔPC1 | B.MGI | ΔMGI | EG.PC1 | ΔPC1 | EG.MGI | ΔMGI |
| NG to Baseline* | NG.PC1 | NA | - | - | - | - | - | - | - | - | - | - | - |
| | ΔPC1 | -0.86 | NA | - | - | - | - | - | - | - | - | - | - |
| | NG.MGI | 0.37 | -0.42 | NA | - | - | - | - | - | - | - | - | - |
| | ΔMGI | -0.36 | **0.40** | -0.98 | NA | - | - | - | - | - | - | - | - |
| Baseline to EG | B.PC1 | 0.28 | _-0.51_ | | | NA | - | - | - | - | - | - | - |
| | ΔPC1 | | | | | | NA | - | - | - | - | - | - |
| | B.MGI | | | | | | | NA | - | - | - | - | - |
| | ΔMGI | | | 0.39 | _-0.38_ | | 0.53 | | NA | - | - | - | - |
| NG to EG | EG.PC1 | 0.4 | -0.44 | | | 0.89 | | | 0.48 | NA | - | - | - |
| | ΔPC1 | -0.56 | _0.43_ | | | 0.49 | | | 0.43 | 0.43 | NA | - | - |
| | EG.MGI | | | 0.39 | -0.37 | 0.53 | | | 1.00 | 0.48 | 0.43 | NA | - |
| | ΔMGI | | | | | 0.44 | | | 0.86 | 0.41 | **0.56** | 0.86 | NA |

EP 2 994 756 B1

19

Table 4

| Spearman correlation (rho) | | NG to Baseline* | | | |
|---|---|---|---|---|---|
| | | NG.PC1 | ΔPC1 | NG.MGI | ΔMGI |
| NG to Baseline* | NG.PC1 | NA | - | - | - |
| | ΔPC1 | -0.43 | NA | - | - |
| | NG.MGI | | | NA | - |
| | ΔMGI | | **0.37** | | NA |

At NG (and also at EG), there is a significant correlation between MGI and PC1-values among the 50 subjects (Spearman correlation NG: *rho*= 0.37, *p*<0.01; EG: *rho*= 0.48, *p<0.001).* Moreover, between NG and Baseline (and also between Baseline and EG), the PC1-values of the 100 microbiota are positively correlated with MGI (Spearman correlation; All: *rho*= 0.74, *p*<0.001; NG-Baseline: *rho*= 0.77, *p*<0.001; Baseline-EG: *rho*= 0.79, *p*<0.001).

PC1-change (ΔPC1) is also clinically relevant. Among the 50 hosts, in each of the two RPM-segments (NG-to-Baseline and Baseline-to-EG), within-subject ΔPC1 and ΔMGI (i.e. MGI-change) are significantly correlated (labeled as **underlined bold),** while the inter-segment ΔPC1-ΔPC1 correlation and inter-phase ΔMGI-ΔMGI correlation are also significant(labeled as *underlined Italic).* Moreover, the within-subject ΔPC1 is significantly correlated with the within-subject ΔMGI between NG and EG (Spearman correlation *rho*=0.56, *p*=0). Interestingly, for the 10 bottom-quintiles subjects with relatively stable MGI NG-to-EG, ΔPC1 is not significantly correlated with that of ΔMGI (Spearman correlation *rho*=0.25, *p*=0.48). However, for the 10 top-quintile subjects where MGI change the most NG-to-EG, ΔMGI is significantly correlated with ΔPC1 (Spearman correlation *rho*=0.64, *p*=0.05), suggesting that ΔPC1 could quantitatively model the degree of alteration of symptoms in gingivitis.

***Two types of hosts with distinct sensitivity to gingivitis*** Among the 50 subjects, most hosts exhibit a largely consistent microbiota structure during the disease progression from NG to EG (see Fig. 8A). Although NG-Baseline (or Baseline-EG) PC1-changes vary considerably among the 50-host cohort, the rate of microbiota change NG-Baseline and that of microbiota change Baseline-EG are largely similar within each subject. The rate of MGI change follows a similar pattern. Furthermore, the gingivitis severity (i.e., MGI) at EG is highly correlated with that at NG, as is microbiota structure (i.e. PC1). The persistence of disease outcome as well as microbiota structure for majority of the hosts in EG (as compared to NG) suggests the presence of host-dependent (and likely personal) factors in determining the susceptibility to gingivitis reoccurrence in natural human populations.

PCA based on within-subject changes of both microbiota (ΔPC1 at NG-to-Baseline and ΔPC1 at Baseline-to-EG) and clinical symptom (ΔMGI at NG-to-Baseline and ΔMGI at Baseline-to-EG) along RPM reveal the divergence of disease susceptibility among the 50 hosts.

As shown in Fig. 8B, all hosts in the 50-member cohort are plotted on the first two principle components of the PCA based on the change profiles of microbiota and MGI. The histogram and the kernel density plot (solid line) describing distribution of the 50 hosts along the principle component of the PCA are shown. The vertical dash line divides the 50 hosts into Type-I (dots) and Type-II (triangles). The four variables as main contributors to these clusters are determined and plotted by their loadings in these two principle components, "a" denotes ΔPC1 (NG-Baseline); "b" denotes ΔMGI (NG-Baseline); "c" denotes ΔPC1(Baseline-EG); and "d" denotes ΔMGI(Baseline-EG).

The distribution pattern of the 50 hosts suggests a bimodal distribution (p = 0.74 for the hypothesis of non-bimodal distribution based on Hartigans' dip test for unimodality), where a discriminating line can be drawn to divide the hosts into two types which the present inventors designate as less gingivitis-sensitive Type-I (17 individuals) and gingivitis-sensitive Type-II (33 individuals). Type-II hosts are characterized by more acute changes in both microbiota structure and MGI than Type-I hosts (see Fig. 8A and Fig. 8C). For an average Type-II host, the PC1-change rate along RPM is 0.33 per day and the MGI-change rate along RPM is 0.05 per day, which are respectively 2.21 fold and 1.89 fold of an average Type-I host (see Fig. 8C).

At both NG and EG, there are significant relationship between gingivitis-sensitive types and the relative abundance of certain taxa (*p*<0.05, Wilcoxon rank-sum test), which include *Abiotrophia, Selenomonas,* uncultured *Lachnospiraceae, Peptococcus,* unclassified *Bacteroidaceae, Peptostreptococcus, Oribacterium* and *Veillonellaceae;* these taxa are all enriched in Type-II hosts as compared to Type I hosts, except *Abiotrophia* which is enriched in Type-I (see Fig. 8D). Most (five) of these Type-II-hosts associated genera are among the 15 PC1-drivers.

Interestingly, compared to Type-I hosts, those genera enriched in Type-II hosts at NG and EG are also of higher abundance in Type-II hosts at their Baseline. Thus the heterogeneity of plaque microbiota among hosts likely can explain at least partially, either as a cause or as a consequence, the inter-host phenotypic variations of gingivitis sensitivity and possibly susceptibility to disease reoccurrence in human populations.

*Microbial Indices of Gingivitis* The strong correlation between PC1 and disease symptom (MGI) both between subjects and within subjects thus suggest PC1 could potentially be used to model disease progression. To test whether predictive models of gingivitis might be built based on plaque microbiota, the 50-host cohort is used as a training set for model construction, while an additional 41 human subjects with naturally occurring gingivitis are recruited and then each sampled at both NG and Baseline (thus 82 additional microbiota samples are sequenced) for model validation.

(1) MiG27: The present inventors derive a "microbial index of gingivitis" (MiG) based on the relative abundance of the 27 bacterial markers that distinguish between the Baseline stage and the gingivitis stages (NG and EG) in the 50-host cohort (MiG27), via the following equation:

$$MiG27 = (\frac{\sum_{i=22} abundance(g_{Gingivtis-enriched})_i}{22} - \frac{\sum_{j=5} abundance(g_{Health-enriched})_j}{5}) \times 10$$

In the 50-host cohort, this index is highly correlated with MGI during both NG-to-Baseline ($p<0.001$, Student's $t$-test) and Baseline-to-EG ($p<0.001$, Student's $t$-test): the area under the receiver operating characteristic (ROC) curve is 99.52% (95% confidence interval: 98.77%-99.52%) at NG-to-Baseline and 99.84% (95% confidence interval: 99.53%-99.84%) at baseline-to-EG.

To validate the predictive power of MiG27, The present inventors predict gingivitis status of the 41 hosts in the new cohort using their NG microbiota. Fig. 9 shows the MiG27 indices of the additional cohort of 41 hosts. Boxes represent the IQR and the lines inside represent the median. Whiskers denote the lowest and highest values within 1.5x IQR. The MiG27 between NG (MGI>1.18) and Baseline (MGI<1.12) is significantly different ($p<0.001$, paired $t$-test, t statistic= 22.3), e.g. the top 27 samples with the highest MiG27 are all correctly classified as gingivitis. The overall accuracy of prediction (based on Linear Discriminant Analysis) for diseased state versus healthy state is 94% (i.e., an error rate of 6.1%) (see Table 5 below). These data suggest that this MiG27 be valuable for screening diseased gingival in clinical settings.

(2) **MiG15:** Although MiG27 distinguishes health and gingivitis with high accuracy, a classifier system for diseased severity in gingivitis population will be valuable. Thus MiG15, which is based on the relative abundance of 15 bacterial genera that drive the structural heterogeneity of microbiota along PC1, is derived. The MiG15 of a given microbiota is calculated via the following equation:

$$MiG15 = (\frac{\sum_{i=13} abundance(g_{High\_PC1-enriched})_i}{13} - \frac{\sum_{j=2} abundance(g_{Low\_PC1-enriched})_j}{2}) \times 10$$

The present inventors then regress the relative PC1-values (**Y**: the development of gingivitis) on MiG15 (**X**) using linear regression. The formula for prediction is: **Y=-0.97-4.62X.** This revised model is able to account for 60% of variance in PC1 location in the 50-host cohort. The predictive power of this model on disease severity is tested based on the NG microbiota in the 41-host cohort. Fig. 10 shows the MiG15 indices of the additional cohort of 41 hosts. Boxes represent the interquartile range (IQR) and the lines inside represent the median. Whiskers denote the lowest and highest values within 1.5x IQR. The heatmap indicates the ability of MiG15 to discriminate healthy and gingivitis status of hosts. MiG15 shows significant correlation with mean MGI for each subjects at NG *(p <0.05*, spearman correlation). Categorization of both predicted values and test values (of PC1) into three quantiles reveals an error rate of prediction at 24.4% (see Table 5 below). Therefore, the MiG15-based model is able to predict the gingivitis severity in human hosts as defined by PC1 at approximately 75% accuracy.

(3) **MiG-Sensitivity (MiG-S):** The present inventors further derive a "microbial index of gingivitis sensitivity" (MiG-S) based on the relative abundance of the eight bacterial markers that distinguish between the Type-I and Type-II in the 50-host cohort at NG (MiG-S), via the following equation:

$$MiG-S = (\frac{\sum_{i=7} abundance(g_{TypeII-enriched})_i}{7} - \frac{\sum_{j=1} abundance(g_{TypeI-enriched})_j}{1}) \times 10$$

In the 50-host cohort, this index is highly correlated with types ($p<0.05$, Wilcoxon rank-sum test): the area under the ROC curve is 74.0% (95% confidence interval: 60.2%-74.0%) (see Fig. 11), suggesting an up to 74.0% accuracy of predicting gingivitis-sensitivity host-types.

Table 5. Predictive Models of Human Gingivitis based on Plaque Microbiota

| | Error rate | MiG27 | MiG15 | MiG-S |
|---|---|---|---|---|
| Clinical status | Health vs. Gingivitis | 6.1% | 6.1% | - |
| Categorized status of | Based on MGI | 41.5% | 41.5% | - |
| gingivitis | Based on PC1 | 24.4% | 24.4% | - |
| Gingivitis sensitivity of the host | Based on change-pattern of PC1 and MGI | - | - | 26.0% |

**Discussion**

The present retrogress-progression model of gingivitis reveals source of the heterogeneity of gingival microbiota both within-subject and in natural populations. The developmental program between the healthier and the more diseased states is primarily driven by 15 bacterial genera, most of which increase in relative abundance (except two which decrease) along the development. The taxonomic shift of microbiota is accompanied by a functional shift: the observed gingivitis-enriched functions such as flagellar biosynthesis might be traced to bacterial oral-mobility, as the flagellar can assist invading host tissues and escaping phagocytosis. Notably, these disease-driving taxa, mostly predominant oral symbionts in normal humans, serve as main hubs of microbial interactions in both natural and experimental gingivitis populations. The evidence thus supports a polymicrobial nature of gingivitis, driven not by a particular pathogen but by overall taxonomic and functional changes driven by specific members of our oral microbial communities.

Two host-types (Type-I and Type-II) with distinct sensitivity/susceptibility to gingivitis are present, with Type-II hosts featuring averagely over two times more acute disease-development than Type-I hosts. Moreover, gingivitis recurrence appears personalized, as the gingivitis severity (e.g., MGI) at EG is highly correlated with that at NG, while the disease progression rate (i.e. Baseline-to-EG) is highly correlated with the disease retrogression rate (i.e. NG-to-Baseline). The present inventors have identified a microbial link to the two host-types, with eight bacterial taxa specifically associated (seven enriched and one depleted) with Type-II hosts at each of NG, Baseline and EG. However, as such association between taxa and host-types actually persists even at Baseline (i.e., "healthy" state), microbial factors likely play prominent roles in host-type formation, and it is possible that Type-II hosts are predisposed to gingivitis reoccurrence due to their residential microbiota at NG.

Furthermore, uncovering these major sources of variation in gingival microbiota might have implications in periodontal diseases. Gingivitis can advance to periodontitis, which is a major cause of tooth loss in adults. However, the role of gingivitis in periodontitis pathogenesis remains controversial: an etiological connection between them has been postulated but not yet proven. One confounding factor has been that not all gingivitis cases proceed into periodontitis: epidemiological studies show that approximately 50% of adults have gingivitis around more than six teeth, while only 15% of adults suffer from periodontitis. In the identified "gingivitis-driver" genera, several species (e.g., *Tannerella forsythensis, Peptostreptococcus micros, Fusobacterium nucleatum subsp., Haemophilus paraphrophilus* and *Capnocytophaga* sp. oral clone CZ006 et. al.) are reportedly associated with periodontitis. In addition, those potential markers of severe gingivitis the present inventors identify (e.g. *Tannerella, Treponema* species and the TM7 phylum) are reportedly enriched in periodontitis. Furthermore, several potential markers of Type II hosts (e.g. *Selenomonas, Peptostreptococcus,* unclassified *Lachnospiraceae, Veillonellaceae* and *Oribacterium*), which exhibit higher disease acuteness and susceptibility to reoccurrence, are found to be enriched in periodontitis. The collective evidence strongly supports a link of severe gingivitis and periodontitis, and also provides a possible explanation of the variation of periodontitis susceptibility in human populations.

Finally, the identified microbial drivers of gingivitis development and susceptibility provide novel opportunities to improve clinical practice. In gingivitis, the gingival tissue exhibits color change color, contour alteration, increased sulcular exudates and bleeding upon provocation. Based on one or more of such host symptoms, current gingival indices proposed or practiced are subjective, prone to human bias and error and difficult to reproduce, as such indices are heavily dependent upon the human examiner's visual observation and individual judgment. For example, despite its prevalent use in clinical practice, MGI in being a subjective measure of gingivitis severity can be of poor reproductivity among different examiners. Moreover, as symptom of gingivitis can vary greatly among different teeth (and even probing points), testing two probing sites for each of the 28 teeth for each patient can be time- and labor-intensive. These drawbacks have collectively confounded cross-examiner and cross-patients analysis of gingivitis. The present inventors develop and validate an alternative and likely complementary measure for gingivitis that is based on quantitative analysis of plaque microbiota. The proposed MiG-based predictive models are able to predict diseased microbiota at 95% accuracy, distinguish different disease-stages with 75% accuracy, and potentially predict disease sensitivity. MiGs can thus serve as more sensitive, reliable and objective measures of gum health and gingivitis susceptibility and thus contribute to the diagnosis, prognosis and intervention of gum diseases.

The potential of human microbiota as venues in tracking and diagnosing host conditions (diseases, diets, etc) is dependent on, and limited by, the degree of heterogeneity in microbiota-condition link at the population level. In the gut, variation of microbiota structure between hosts appears to dominate variation among conditions (e.g. lean or obese, or on a normal or high-fat diet). However, the results here reveal that the opposite appears to be true for oral microbiota, and that differences between healthy and diseased oral microbiota within a subject are larger than inter-personal differences. Although the mechanism for this difference in response sizes in microbial communities within different body habitats is unknown, the present invention suggests that the oral microbiota might offer certain advantages as biomarkers for oral, and perhaps even systemic, diseases.

Unless otherwise indicated, all percentages, ratios, and proportions are calculated based on weight of the total composition. All temperatures are in degrees Celsius (°C) unless otherwise indicated. All measurements made are at 25°C, unless otherwise designated. All component or composition levels are in reference to the active level of that component or composition, and are exclusive of impurities, for example, residual solvents or by-products, which may be present in commercially available sources.

It should be understood that every maximum numerical limitation given throughout this specification includes every lower numerical limitation, as if such lower numerical limitations are expressly written herein. Every minimum numerical limitation given throughout this specification will include every higher numerical limitation, as if such higher numerical limitations are expressly written herein. Every numerical range given throughout this specification will include every narrower numerical range that falls within such broader numerical range, as if such narrower numerical ranges are all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

Every document cited herein, including any cross referenced or related patent or application is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

**Claims**

1. A method of identifying a biomarker indicative of a subject mammal's condition, comprising the steps:

    a) selecting a first set of test mammals having a first disease;
    b) obtaining a first oral sample containing a first microbial community from each of the first set of test mammals having the first disease, wherein the first microbial community comprises one or more microbial types;
    c) treating each of the first set of test mammals having the first disease, who have been first oral sampled, so as to eliminate or reduce the first disease;
    d) obtaining a second oral sample containing a second microbial community from each of the first set of test mammals who have been treated, wherein the second microbial community comprises one or more microbial types;
    e) making the first disease reoccur in each of the first set of test mammals who have been second oral sampled;
    f) obtaining a third oral sample containing a third microbial community from each of the first set of test mammals in whom the first disease has reoccurred, wherein the third microbial community comprises one or more microbial types;
    g) measuring the first, second and third oral samples to obtain abundances of the one or more microbial types in the first, second and third microbial communities, respectively;
    h) statistically analyzing the obtained abundances of the one or more microbial types in the first, second and third microbial communities across the first set of test mammals to identify those microbial types whose abundances correlate with a statistical significance to a condition of the first set of test mammals as a first group of microbial types, wherein the condition is selected from the group consisting of: presence of the first disease, severity of the first disease, sensitivity to the first disease, and combinations thereof;

i) selecting one or more microbial types from the first group of microbial types as the biomarker indicative of said subject mammal's condition.

2. The method according to claim 1, wherein in step h), the obtained abundances of the one or more microbial types in the first, second and third microbial communities are statistically analyzed by a pair-wise comparative analysis or a multivariate analysis.

3. The method according to claim 2, wherein the multivariate analysis is selected from the group consisting of principal component analysis, principal coordinate analysis, correspondence analysis, detrended correspondence analysis, cluster analysis, discriminant analysis, canonical discriminant analysis, and combinations thereof, preferably principal component analysis.

4. The method according to claim 2, wherein in step h), the obtained abundances of the one or more microbial types in the first, second and third microbial communities are statistically analyzed by a pair-wise comparative analysis comprising the steps:

1) comparing said first microbial community and said second microbial community of each of the first set of test mammals to determine change in the obtained abundances of each microbial type between said first microbial community and said second microbial community;

2) comparing the change in the obtained abundances of each microbial type from step 1) across the first set of test mammals to select those microbial types that exhibit statistically significant changes in abundances as a primary group of microbial types;

3) comparing said second microbial community and said third microbial community of each of the first set of test mammals to determine change in the obtained abundances of each microbial type between said second microbial community and said third microbial community;

4) comparing the change in the obtained abundances of each microbial type from step 3) across the first set of test mammals to select those microbial types that exhibit statistically significant changes in abundances as a secondary group of microbial types; and

5) comparing the primary group of microbial types and the secondary group of microbial types to identify those overlapped microbial types as the first group of microbial types.

5. The method according to claim 2, wherein in step h), the obtained abundances of the one or more microbial types in the first, second and third microbial communities are statistically analyzed by a multivariate analysis comprising the steps:

1) orthogonally transforming the obtained abundances of the one or more microbial types in the first, second and third microbial communities to derive a vector accounting for the largest variance among the obtained abundances; and

2) identifying those microbial types with the obtained abundances that exhibit statistically significant correlations to the derived vector as the first group of microbial types.

6. The method according to claim 2, wherein in step h), the obtained abundances of the one or more microbial types in the first, second and third microbial communities are statistically analyzed by a multivariate analysis comprising the steps:

1) orthogonally transforming the obtained abundances of the one or more microbial types in the first, second and third microbial communities to derive a vector accounting for the largest variance among the obtained abundances;

2) projecting the obtained abundances of the one or more microbial types in each of the first, second and third microbial communities of each of the first set of test mammals on the derived vector to obtain a projection value for each of the first, second and third microbial communities of each of the first set of test mammals;

3) calculating a change rate of the projection values across the first, second and third microbial communities for each of the first set of test mammals;

4) classifying the first set of test mammals, based on the calculated change rates, into a first subset of test mammals and a second subset of test mammals, wherein the first subset of test mammals exhibit greater change rates than the second subset of test mammals; and

5) comparing the first, second and third microbial communities of the first subset of test mammals with the first, second and third microbial communities of the second subset of test mammals, respectively, to identify those microbial types whose abundances in each of the first, second and third microbial communities are statistically significantly different between the first subset of test mammals and the second subset of test mammals, as the first group of microbial types.

7. The method according to claim 1, wherein the first disease is a microbe-related disease.

8. The method according to claim 7, wherein the microbe-related disease is selected from the group consisting of gingivitis, periodontitis, dental caries, halitosis, oral ulcer, and any combination thereof, and preferably gingivitis.

9. The method according to claim 1, further comprising the steps:

1) selecting a second set of test mammals having a second disease;
2) repeating steps b) to h) to identify a second group of microbial types;
3) comparing the first group of microbial types and the second group of microbial types to identify those overlapped microbial types as a subgroup of microbial types; and
4) selecting one or more microbial types from said subgroup of microbial types as the biomarker indicative of said subject mammal's condition, wherein the condition is selected from the group consisting of: presence of the first disease and the second disease, severity of the first disease and the second disease, sensitivity to the first disease and the second disease, and combinations thereof.

10. The method according to claim 1, wherein the microbial type is selected from the group consisting of taxonomic categories of a bacterium, functional categories of a microbe, and combinations thereof.

11. The method according to claim 10, wherein the microbial type is selected from the group consisting of a bacterial phylum, a bacterial class, a bacterial family, a bacterial order, a bacterial genus, a bacterial species, a functional gene of a microbe, a gene ortholog group of a microbe, a motif of peptide or protein of a microbe, a conserved peptide or protein domain of a microbe, a none-coding nucleotide sequence of a microbe, and combinations thereof, preferably a bacterial genus.

12. The method according to claim 1, wherein the first, second and third oral samples are selected from the group consisting of a salivary sample, a supragingival plaque sample, a subgingival plaque sample, a tooth plaque sample, and combinations thereof.

13. The method according to claim 1, wherein in step g), the first, second and third oral samples are measured by a method selecting from the group consisting of 16S rRNA analysis, metagenomics, and combination thereof.

14. The method according to claim 1, wherein the statistical significance has a level of $p<0.05$, preferably $p<0.01$, and more preferably $p<0.001$.

15. A computer-aided system for performing the method according to anyone of claims 1 to 14 comprising

a) a sampling section for sampling

1) a first oral sample containing a first microbial community from each of a set of test mammals having a disease, wherein the first microbial community comprises one or more microbial types,
2) a second oral sample containing a second microbial community from each of the set of test mammals who have been treated to eliminate or reduce the disease, wherein the second microbial community comprises one or more microbial types, and
3) a third oral sample containing a third microbial community from each of the set of test mammals in whom the disease has reoccurred, wherein the third microbial community comprises one or more microbial types;

b) a measuring section in communication with the sampling section, wherein the measuring section is configured for measuring the first, second and third oral samples to obtain abundances of the one or more microbial types in the first, second and third microbial communities, respectively; and

c) a computing section in communication with the measuring section, wherein the computing section is configured for receiving and statistically analyzing the obtained abundances of the one or more microbial types in the first, second and third microbial communities across the set of test mammals to identify those microbial types whose abundances correlate with a statistical significance to a condition of the set of test mammals as the biomarker indicative of said subject mammal's condition,

wherein the condition is selected from the group consisting of: presence of the disease, severity of the disease, sensitivity to the disease, and combinations thereof.

**Patentansprüche**

1. Verfahren zum Identifizieren eines Biomarkers, der den Zustand eines Säugetiersubjekts angibt, umfassend die Schritte:

a) Auswählen einer ersten Gruppe von Testsäugetieren, die eine erste Erkrankung aufweisen;
b) Erhalten einer ersten oralen Probe, die eine erste Mikrobengemeinschaft von jedem der ersten Gruppe von Testsäugetieren mit der ersten Erkrankung enthält, wobei die erste Mikrobengemeinschaft einen oder mehrere mikrobielle Typen umfasst;
c) Behandeln von jedem der ersten Gruppe von Testsäugetieren, die die erste Erkrankung aufweisen und die als erste orale Probe ausgewählt worden sind, um die erste Erkrankung zu eliminieren oder zu reduzieren;
d) Erhalten einer zweiten oralen Probe, die eine zweite Mikrobengemeinschaft von jedem der ersten Gruppe von behandelten Testsäugetieren enthält, wobei die zweite Mikrobengemeinschaft einen oder mehrere mikrobielle Typen umfasst;
e) Verursachen des erneuten Auftretens der ersten Erkrankung in jedem der ersten Gruppe von Testsäugetieren, die als zweite orale Probe ausgewählt worden sind;
f) Erhalten einer dritten oralen Probe, die eine dritte Mikrobengemeinschaft von jedem der ersten Gruppe von Testsäugetieren enthält, bei denen die erste Erkrankung erneut aufgetreten ist, wobei die dritte Mikrobengemeinschaft einen oder mehrere mikrobielle Typen umfasst;
g) Messen der ersten, zweiten und dritten Proben, um Abundanzen des einen oder der mehreren mikrobiellen Typen in der ersten, zweiten bzw. dritten Mikrobengemeinschaft zu erhalten;
h) statistisches Analysieren der erhaltenen Abundanzen des einen oder der mehreren mikrobiellen Typen in der ersten, zweiten und dritten Mikrobengemeinschaft in der ersten Gruppe von Testsäugetieren, um diejenigen mikrobiellen Typen, deren Abundanzen mit einer statistischen Signifikanz für eine Bedingung der ersten Gruppe von Testsäugetieren korrelieren, zu identifizieren als eine erste Gruppe von mikrobiellen Typen, wobei die Bedingung ausgewählt ist aus der Gruppe bestehend aus: Vorliegen der ersten Erkrankung, Schweregrad der ersten Erkrankung, Anfälligkeit für die erste Erkrankung und Kombinationen daraus;
i) Auswahl von einem oder mehreren mikrobiellen Typen aus der ersten Gruppe von mikrobiellen Typen als Biomarker zur Angabe des Zustands des Säugetiersubjekts.

2. Verfahren nach Anspruch 1, wobei in Schritt h) die erhaltenen Abundanzen des einen oder der mehreren mikrobiellen Typen in der ersten, zweiten und dritten Mikrobengemeinschaft durch eine paarweise Vergleichsanalyse oder eine multivariate Analyse statistisch analysiert werden.

3. Verfahren nach Anspruch 2, wobei die multivariate Analyse ausgewählt ist aus der Gruppe bestehend aus Hauptkomponentenanalyse, Hauptkoordinatenanalyse, Korrespondenzanalyse, trendbereinigte Korrespondenzanalyse, Cluster-Analyse, Diskriminanzanalyse, kanonische Diskriminanzanalyse und Kombinationen davon, vorzugsweise Hauptkomponentenanalyse.

4. Verfahren nach Anspruch 2, wobei in Schritt h) die erhaltenen Abundanzen des einen oder der mehreren mikrobiellen Typen in der ersten, zweiten und dritten Mikrobengemeinschaft durch eine paarweise Vergleichsanalyse statistisch analysiert werden, umfassend die Schritte:

1) Vergleichen der ersten Mikrobengemeinschaft und der zweiten Mikrobengemeinschaft von jedem der ersten Gruppe von Testsäugetieren, um die Veränderung in den erhaltenen Abundanzen jedes mikrobiellen Typs zwischen der ersten Mikrobengemeinschaft und der zweiten Mikrobengemeinschaft zu bestimmen;
2) Vergleichen der Veränderung der erhaltenen Abundanzen jedes mikrobiellen Typs aus Schritt 1) über der ersten Gruppe von Testsäugetieren zur Auswahl derjenigen mikrobiellen Typen, die statistisch signifikante Veränderungen bei Abundanzen aufweisen, als primäre Gruppe von mikrobiellen Typen;
3) Vergleichen der zweiten Mikrobengemeinschaft und der dritten Mikrobengemeinschaft von jedem der ersten Gruppe von Testsäugetieren, um die Veränderung in den erhaltenen Abundanzen jedes mikrobiellen Typs

EP 2 994 756 B1

zwischen der zweiten Mikrobengemeinschaft und der dritten Mikrobengemeinschaft zu bestimmen;
4) Vergleichen der Veränderung der erhaltenen Abundanzen jedes mikrobiellen Typs aus Schritt 3) in der gesamten ersten Gruppe von Testsäugetieren zur Auswahl derjenigen mikrobiellen Typen, die statistisch signifikante Veränderungen bei Abundanzen aufweisen, als sekundäre Gruppe von mikrobiellen Typen; und
5) Vergleichen der primären Gruppe von mikrobiellen Typen und der sekundären Gruppe von mikrobiellen Typen, um diejenigen mikrobiellen Typen, die sich überlappen, als erste Gruppe von mikrobiellen Typen zu identifizieren.

5. Verfahren nach Anspruch 2, wobei in Schritt h) die erhaltenen Abundanzen des einen oder der mehreren mikrobiellen Typen in der ersten, zweiten und dritten Mikrobengemeinschaft durch eine multivariate Analyse statistisch analysiert werden, umfassend die Schritte:

1) orthogonale Transformation der erhaltenen Abundanzen des einen oder der mehreren mikrobiellen Typen in der ersten, zweiten und dritten Mikrobengemeinschaft, um einen Vektor abzuleiten, der die größte Varianz unter den erhaltenen Abundanzen darstellt; und
2) Identifizieren derjenigen mikrobiellen Typen mit den erhaltenen Abundanzen, die statistisch signifikante Korrelationen zum abgeleiteten Vektor aufweisen, als erste Gruppe mikrobieller Typen.

6. Verfahren nach Anspruch 2, wobei in Schritt h) die erhaltenen Abundanzen des einen oder der mehreren mikrobiellen Typen in der ersten, zweiten und dritten Mikrobengemeinschaft durch eine multivariate Analyse statistisch analysiert werden, umfassend die Schritte:

1) orthogonale Transformation der erhaltenen Abundanzen des einen oder der mehreren mikrobiellen Typen in der ersten, zweiten und dritten Mikrobengemeinschaft, um einen Vektor abzuleiten, der die größte Varianz unter den erhaltenen Abundanzen darstellt;
2) Projizieren der erhaltenen Abundanzen des einen oder der mehreren mikrobiellen Typen in jeder der ersten, zweiten und dritten Mikrobengemeinschaft jedes der ersten Gruppe von Testsäugetieren auf den abgeleiteten Vektor, um für jede der ersten, zweiten und dritten Mikrobengemeinschaft von jedem der ersten Gruppe von Testsäugetieren einen Projektionswert zu erhalten;
3) Berechnen einer Änderungsrate der Projektionswerte über der ersten, zweiten und dritten Mikrobengemeinschaft für jedes der ersten Gruppe von Testsäugetieren;
4) Klassifizieren der ersten Gruppe von Testsäugetieren, basierend auf den berechneten Änderungsraten, in eine erste Untergruppe von Testsäugetieren und eine zweite Untergruppe von Testsäugetieren, wobei die erste Untergruppe von Testsäugetieren größere Änderungsraten aufweist als die zweite Untergruppe von Testsäugetieren; und
5) Vergleichen der ersten, zweiten und dritten Mikrobengemeinschaft der ersten Untergruppe von Testsäugetieren mit der ersten, zweiten bzw. dritten Mikrobengemeinschaft der zweiten Untergruppe von Testsäugetieren, um diejenigen mikrobiellen Typen, deren Abundanzen in jeder der ersten, zweiten und dritten Mikrobengemeinschaft zwischen der ersten Untergruppe von Testsäugetieren und der zweiten Untergruppe von Testsäugetieren statistisch signifikant unterschiedlich sind, als erste Gruppe von mikrobiellen Typen zu identifizieren.

7. Verfahren nach Anspruch 1, wobei die erste Erkrankung eine mikrobenbezogene Erkrankung ist.

8. Verfahren nach Anspruch 7, wobei die mikrobenbezogene Erkrankung ausgewählt ist aus der Gruppe bestehend aus Gingivitis, Parodontitis, Zahnkaries, Halitosis, Mundgeschwüren, und beliebigen Kombinationen daraus, und vorzugsweise Gingivitis ist.

9. Verfahren nach Anspruch 1, ferner umfassend die Schritte:

1) Auswählen einer zweiten Gruppe von Testsäugetieren, die eine zweite Erkrankung aufweisen;
2) Wiederholen der Schritte b) bis h) zum Identifizieren einer zweiten Gruppe von mikrobiellen Typen;
3) Vergleichen der ersten Gruppe von mikrobiellen Typen und der zweiten Gruppe von mikrobiellen Typen, um diejenigen mikrobiellen Typen, die sich überlappen, als eine Untergruppe von mikrobiellen Typen zu identifizieren; und
4) Auswählen von einem oder mehreren mikrobieller Typen aus der Untergruppe von mikrobiellen Typen als Biomarker, der den Zustand des betreffenden Säugetiersubjekts anzeigt, wobei der Zustand ausgewählt ist aus der Gruppe bestehend aus: Vorliegen der ersten Erkrankung und der zweiten Erkrankung, Schweregrad der ersten Erkrankung und der zweiten Erkrankung, Anfälligkeit gegenüber der ersten Erkrankung und der zweiten

27

Erkrankung und Kombinationen daraus.

10. Verfahren nach Anspruch 1, wobei der mikrobielle Typ ausgewählt ist aus der Gruppe bestehend aus taxonomischen Kategorien eines Bakteriums, funktionellen Kategorien einer Mikrobe und Kombinationen davon.

11. Verfahren nach Anspruch 10, wobei der mikrobielle Typ ausgewählt ist aus der Gruppe bestehend aus einem Bakterienstamm, einer Bakterienklasse, einer Bakterienfamilie, einer Bakterienordnung, einer Bakteriengattung, einer Bakterienspezies, einem funktionellen Gen einer Mikrobe, einer genorthologen Gruppe einer Mikrobe, einem Peptid- oder Protein-Motiv einer Mikrobe, einer konservierten Peptid- oder Proteindomäne einer Mikrobe, einer nicht codierenden Nukleotidsequenz einer Mikrobe, und Kombinationen daraus, und wobei er vorzugsweise eine Bakteriengattung ist.

12. Verfahren nach Anspruch 1, wobei die erste, zweite und dritte orale Probe ausgewählt ist aus der Gruppe bestehend aus einer Speichelprobe, einer supragingivalen Plaque-Probe, einer subgingivalen Plaque-Probe, einer Zahnbelagsprobe und Kombinationen daraus.

13. Verfahren nach Anspruch 1, wobei in Schritt g) die ersten, zweiten und dritten oralen Proben anhand eines Verfahrens gemessen werden, das ausgewählt ist aus der Gruppe bestehend aus 16S rRNA-Analyse, Metagenomik und Kombinationen davon.

14. Verfahren nach Anspruch 1, wobei die statistische Signifikanz ein Niveau von $p<0,05$ aufweist, vorzugsweise $p<0,01$ und stärker bevorzugt $p<0,001$.

15. Computerunterstütztes System zur Durchführung des Verfahrens nach einem der Ansprüche 1 bis 14, umfassend

   a) einen Probenahmeabschnitt zur Probenentnahme

   1) einer ersten oralen Probe, die eine erste Mikrobengemeinschaft von jedem einer Gruppe von Testsäugetieren mit einer Erkrankung enthält, wobei die erste Mikrobengemeinschaft einen oder mehrere mikrobielle Typen umfasst,
   2) einer zweiten oralen Probe, die eine zweite Mikrobengemeinschaft von jedem der Gruppe von Testsäugetieren enthält, die zur Eliminierung oder Reduzierung der Erkrankung behandelt worden sind, wobei die zweite Mikrobengemeinschaft einen oder mehrere mikrobielle Typen umfasst, und
   3) einer dritten oralen Probe, die eine dritte Mikrobengemeinschaft von jedem der Gruppe von Testsäugetieren enthält, bei denen die Erkrankung erneut aufgetreten ist, wobei die dritte Mikrobengemeinschaft einen oder mehrere mikrobielle Typen umfasst;

   b) einen Messbereich in Kommunikation mit dem Probenahmeabschnitt, wobei der Messbereich zum Messen der ersten, zweiten und dritten oralen Proben eingerichtet ist, um Abundanzen des einen oder der mehreren mikrobiellen Typen in der ersten, zweiten bzw. dritten Mikrobengemeinschaft zu erhalten; und
   c) einen mit dem Messbereich in Kommunikation befindlichen Berechnungsabschnitt, wobei der Berechnungsabschnitt für den Empfang und die statistische Analyse der erhaltenen Abundanzen des einen oder der mehreren mikrobiellen Typen in der ersten, zweiten und dritten Mikrobengemeinschaft der gesamten Gruppe von Testsäugetieren eingerichtet ist, um diejenigen mikrobiellen Typen, deren Abundanzen mit einer statistischen Signifikanz für einen Zustand der Gruppe von Testsäugetieren korrelieren, als Biomarker zu identifizieren, der den Zustand des Säugetiersubjekts angibt,

   wobei der Zustand ausgewählt ist aus der Gruppe bestehend aus: Vorliegen der Erkrankung, Schweregrad der Erkrankung, Anfälligkeit gegenüber der Erkrankung und Kombinationen daraus.

**Revendications**

1. Procédé d'identification d'un biomarqueur caractéristique d'une condition d'un mammifère sujet, comprenant les étapes consistant à :

   a) sélectionner un premier ensemble de mammifères de test souffrant d'une première maladie ;
   b) obtenir un premier échantillon oral contenant une première communauté microbienne à partir de chacun parmi le premier ensemble de mammifères de test souffrant de la première maladie, dans lequel la première communauté microbienne comprend un ou plusieurs types de microbes ;

c) traiter chacun parmi le premier ensemble de mammifères de test souffrant de la première maladie, dont on a prélevé un premier échantillon oral, de façon à éliminer ou réduire la première maladie ;

d) obtenir un deuxième échantillon oral contenant une deuxième communauté microbienne à partir de chacun parmi le premier ensemble de mammifères de test qui ont été traités, dans lequel la deuxième communauté microbienne comprend un ou plusieurs types de microbes ;

e) faire réapparaître la première maladie dans chacun parmi le premier ensemble de mammifères de test dont on a prélevé un deuxième échantillon oral ;

f) obtenir un troisième échantillon oral contenant une troisième communauté microbienne à partir de chacun parmi le premier ensemble de mammifères de test chez qui la première maladie est réapparue, dans lequel la troisième communauté microbienne comprend un ou plusieurs types de microbes ;

g) mesurer les premier, deuxième et troisième échantillons oraux pour obtenir les abondances du ou des types de microbes dans les première, deuxième et troisième communautés microbiennes, respectivement ;

h) analyser statistiquement les abondances obtenues du ou des types de microbes dans les première, deuxième et troisième communautés microbiennes à travers le premier ensemble de mammifères de test pour identifier ces types de microbes dont les abondances se corrèlent avec une signification statistique à une condition du premier ensemble de mammifères de test en tant que premier groupe de types de microbes, dans lequel la condition est choisie dans le groupe constitué de : présence de la première maladie, gravité de la première maladie, sensibilité à la première maladie, et leurs combinaisons ;

i) sélectionner un ou plusieurs types de microbes du premier groupe de types de microbes en tant que biomarqueur caractéristique de ladite condition du mammifère sujet.

2. Procédé selon la revendication 1, dans lequel, à l'étape h), les abondances obtenues du ou des types de microbes dans les première, deuxième et troisième communautés microbiennes sont analysées statistiquement par une analyse comparative par paire ou une analyse à variables multiples.

3. Procédé selon la revendication 2, dans lequel l'analyse à variables multiples est choisie dans le groupe constitué d'analyse en composantes principales, analyse en coordonnées principales, analyse de correspondance, analyse de correspondance redressée, analyse typologique, analyse discriminante, analyse discriminante canonique, et leurs combinaisons, de préférence une analyse en composantes principales.

4. Procédé selon la revendication 2, dans lequel, à l'étape h), les abondances obtenues du ou des types de microbes dans les première, deuxième et troisième communautés microbiennes sont analysées statistiquement par une analyse comparative par paire comprenant les étapes consistant à :

1) comparer ladite première communauté microbienne et ladite deuxième communauté microbienne de chacun parmi le premier ensemble de mammifères de test pour déterminer un changement dans les abondances obtenues de chaque type de microbe entre ladite première communauté microbienne et ladite deuxième communauté microbienne ;

2) comparer le changement dans les abondances obtenues de chaque type de microbe provenant de l'étape 1) à travers le premier ensemble de mammifères de test pour sélectionner ces types de microbes qui présentent des changements statistiquement significatifs d'abondances en tant que groupe primaire de types de microbes ;

3) comparer ladite deuxième communauté microbienne et ladite troisième communauté microbienne de chacun parmi le premier ensemble de mammifères de test pour déterminer un changement dans les abondances obtenues de chaque type de microbe entre ladite deuxième communauté microbienne et ladite troisième communauté microbienne ;

4) comparer le changement dans les abondances obtenues de chaque type de microbe provenant de l'étape 3) à travers le premier ensemble de mammifères de test pour sélectionner ces types de microbes qui présentent des changements statistiquement significatifs d'abondances en tant que groupe secondaire de types de microbes ; et

5) comparer le groupe primaire de types de microbes et le groupe secondaire de types de microbes pour identifier ces types de microbes en chevauchement en tant que premier groupe de types de microbes.

5. Procédé selon la revendication 2, dans lequel, à l'étape h), les abondances obtenues du ou des types de microbes dans les première, deuxième et troisième communautés microbiennes sont analysées statistiquement par une analyse à variables multiples comprenant les étapes consistant à :

1) transformer orthogonalement les abondances obtenues du ou des types de microbes dans les première, deuxième et troisième communautés microbiennes pour dériver un vecteur représentant la plus grande variance parmi les abondances obtenues ; et

2) identifier ces types de microbes avec les abondances obtenues qui présentent des corrélations statistiquement significatives avec le vecteur dérivé en tant que premier groupe de types de microbes.

6. Procédé selon la revendication 2, dans lequel, à l'étape h), les abondances obtenues du ou des types de microbes dans les première, deuxième et troisième communautés microbiennes sont analysées statistiquement par une analyse à variables multiples comprenant les étapes consistant à :

1) transformer orthogonalement les abondances obtenues du ou des types de microbes dans les première, deuxième et troisième communautés microbiennes pour dériver un vecteur représentant la plus grande variance parmi les abondances obtenues ;

2) projeter les abondances obtenues du ou des types de microbes dans chacune des première, deuxième et troisième communautés microbiennes de chacun parmi le premier ensemble de mammifères de test sur le vecteur dérivé pour obtenir une valeur de projection pour chacune des première, deuxième et troisième communautés microbiennes de chacun parmi le premier ensemble de mammifères de test ;

3) calculer un taux de changement des valeurs de projection à travers les première, deuxième et troisième communautés microbiennes pour chacun parmi le premier ensemble de mammifères de test ;

4) classer le premier ensemble de mammifères de test, sur la base des taux de changement calculés, en un premier sous-ensemble de mammifères de test et un deuxième sous-ensemble de mammifères de test, dans lequel le premier sous-ensemble de mammifères de test présente des taux de changement plus grands que le deuxième sous-ensemble de mammifères de test ; et

5) comparer les première, deuxième et troisième communautés microbiennes du premier sous-ensemble de mammifères de test aux première, deuxième et troisième communautés microbiennes du deuxième sous-ensemble de mammifères de test, respectivement, pour identifier ces types de microbes dont les abondances dans chacune des première, deuxième et troisième communautés microbiennes sont significativement différentes statistiquement entre le premier sous-ensemble de mammifères de test et le deuxième sous-ensemble de mammifères de test, en tant que premier groupe de types de microbes.

7. Procédé selon la revendication 1, dans lequel la première maladie est une maladie liée à un microbe.

8. Procédé selon la revendication 7, dans lequel la maladie liée à un microbe est choisie dans le groupe constitué de gingivite, parodontite, caries dentaires, halitose, ulcère buccal, et n'importe quelle combinaison de ceux-ci, et de préférence la gingivite.

9. Procédé selon la revendication 1, comprenant en outre les étapes suivantes :

1) sélection d'un deuxième ensemble de mammifères de test souffrant d'une deuxième maladie ;

2) répétition des étapes b) à h) pour identifier un deuxième groupe de types de microbes ;

3) comparaison du premier groupe de types de microbes et du deuxième groupe de types de microbes pour identifier les types de microbes en chevauchement en tant que sous-groupe de types de microbes ; et

4) sélection d'un ou plusieurs types de microbes parmi ledit sous-groupe de types de microbes en tant que biomarqueur caractéristique de ladite condition du mammifère sujet, dans lequel la condition est choisie dans le groupe constitué de : présence de la première maladie et de la deuxième maladie, gravité de la première maladie et de la deuxième maladie, sensibilité à la première maladie et à la deuxième maladie, et leurs combinaisons.

10. Procédé selon la revendication 1, dans lequel le type de microbe est choisi dans le groupe constitué de catégories taxonomiques d'une bactérie, catégories fonctionnelles d'un microbe, et leurs combinaisons.

11. Procédé selon la revendication 10, dans lequel le type de microbe est choisi dans le groupe constitué d'un phylum bactérien, une classe bactérienne, une famille bactérienne, un ordre bactérien, un genre bactérien, une espèce bactérienne, un gène fonctionnel d'un microbe, un groupe d'orthologues géniques d'un microbe, un motif de peptide ou de protéine d'un microbe, un domaine peptidique ou protéinique conservé d'un microbe, une séquence nucléotidique non codante d'un microbe, et leurs combinaisons, de préférence un genre bactérien.

12. Procédé selon la revendication 1, dans lequel les premier, deuxième et troisième échantillons oraux sont choisis dans le groupe constitué d'un échantillon salivaire, un échantillon de plaque supragingivale, un échantillon de plaque subgingivale, un échantillon de plaque dentaire, et leurs combinaisons.

13. Procédé selon la revendication 1, dans lequel, à l'étape g), les premier, deuxième et troisième échantillons oraux sont mesurés par un procédé choisi dans le groupe constitué d'analyse d'ARN ribosomique 16S, métagénomique, et une combinaison de ceux-ci.

14. Procédé selon la revendication 1, dans lequel la signification statistique a un niveau de $p < 0,05$, de préférence $p < 0,01$, et plus préférablement $p < 0,001$.

15. Système assisté par ordinateur pour l'exécution du procédé selon l'une quelconque des revendications 1 à 14, comprenant

a) une section d'échantillonnage pour échantillonner

1) un premier échantillon oral contenant une première communauté microbienne à partir de chacun parmi un ensemble de mammifères de test souffrant d'une maladie, dans lequel la première communauté microbienne comprend un ou plusieurs types de microbes,
2) un deuxième échantillon oral contenant une deuxième communauté microbienne à partir de chacun parmi l'ensemble de mammifères de test qui a été traité pour éliminer ou réduire la maladie, dans lequel la deuxième communauté microbienne comprend un ou plusieurs types de microbes, et
3) un troisième échantillon oral contenant une troisième communauté microbienne à partir de chacun parmi l'ensemble de mammifères de test chez qui la maladie est réapparue, dans lequel la troisième communauté microbienne comprend un ou plusieurs types de microbes ;

b) une section de mesure en communication avec la section d'échantillonnage, dans lequel la section de mesure est configurée pour mesurer les premier, deuxième et troisième échantillons oraux pour obtenir les abondances du ou des types de microbes dans les première, deuxième et troisième communautés microbiennes, respectivement ; et

c) une section de calcul en communication avec la section de mesure, dans lequel la section de calcul est configurée pour recevoir et analyser statistiquement les abondances obtenues du ou des types de microbes dans les première, deuxième et troisième communautés microbiennes à travers l'ensemble de mammifères de test pour identifier ces types de microbes dont les abondances se corrèlent avec une signification statistique à une condition de l'ensemble de mammifères de test en tant que biomarqueur caractéristique de ladite condition du mammifère sujet,

dans lequel la condition est choisie dans le groupe constitué de : présence de la maladie, gravité de la maladie, sensibilité à la maladie, et leurs combinaisons.

Fig. 1A

| | Oral Hygiene Phase | | | Induced Gingivitis Phase | | | | Recovery |
|---|---|---|---|---|---|---|---|---|
| Time points | -21 (Day) | -14 | -7 | 0 | 7 | 14 | 21 | >21 (day) |
| Dental Prophylaxis | √ (BOP>2) | √ (BOP>2) | √ (BOP>2) | | | | | √ (BOP>2) |
| MGI record | √ | √ | √ | √ | √ | √ | √ | |
| Plaque collection | √ | | | √ | | | √ | |
| Oral Hygiene | From -21 to -1 day, twice monitored brushing and flossing per day | | | From 0 to 21 day, only twice mouth rinsing with water after meals per day | | | | Monitored oral hygiene |

NG*  Baseline  EG*

Fig. 1B

# Study pipeline

Subject recruiting and Clinical study execution

Measure gingival index and collect two quadrants (1, 3 or 2, 4) supra-gingival plaque from subject

Plaque DNA extraction

Amplification of 16s rDNA sequences by polymerase chain reaction

DNA Sequencing of 16s rDNA amplicons

Whole DNA sequencing

Bioinformatics analysis

Statistic analysis

Fig. 2

Fig. 3

Fig. 4A

Fig. 4B

Fig. 5A

Fig. 5B

NG

Fig. 6A

Baseline

Fig. 6B

41

EG

Fig. 6C

Fig. 7A

Fig. 7B

**C**  (Flagellar assembly)

Fig. 7C

Fig. 8A

Fig. 8B

Fig. 8C

Fig. 8D

Fig. 9

Fig. 10

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102517392 A

- WO 2011011094 A1

### Non-patent literature cited in the description

- **LOUKOPOULOS P ; THORNTON JR ; ROBINSON WF.** Clinical and pathologic relevance of p53 index in canine osseous tumors. *Vet. Pathol.,* May 2003, vol. 40 (3), 237-48
- **LOUKOPOULOS P ; MUNGALL BA ; STRAW RC ; THORNTON JR ; ROBINSON WF.** Matrix metallo-proteinase-2 and -9 involvement in canine tumors. *Vet. Pathol.,* July 2003, vol. 40 (4), 382-94
- **ANA PAULA V. COLOMBO et al.** Impact of Periodontal Therapy on the Subgingival Microbiota of Severe Periodontitis: Comparison Between Good Responders and Individuals With Refractory Periodontitis Using the Human Oral Microbe Identification Microarrray. *Journal of Periodontology,* 01 October 2012, vol. 83 (10), 1279-1287
- **RAVEL J et al.** Vaginal microbiome of reproductive-age women. *Proc. Natl. Acad. Sci. U. S. A.,* 2011, vol. 108, 4680-4687

- **DRAY S ; DUFOUR AB.** The ade4 package: Implementing the duality diagram for ecologists. *Journal of Statistical Software,* 2007, vol. 22 (4), 1-20
- **EDGAR RC.** Search and clustering orders of magnitude faster than BLAST. *Bioinformatics,* 2010, vol. 26 (19), 2460-2461
- **GRIFFEN AL et al.** CORE: a phylogenetically-curated 16S rDNA database of the core oral microbiome. *PLoS One,* 2011, vol. 6 (4), e19051
- **HAMADY M ; LOZUPONE C ; KNIGHT R.** Fast UniFrac: facilitating high-throughput phylogenetic analyses of microbial communities including analysis of pyrosequencing and PhyloChip data. *ISME J,* 2010, vol. 4 (1), 17-27
- **SCHLOSS PD ; GEVERS D ; WESTCOTT SL.** Reducing the effects of PCR (Polymerase Chain Reaction) amplification and sequencing artifacts on 16S rRNA-based studies. *PLoS One,* 2011, vol. 6 (12), e27310